# EUROPEAN PATENT APPLICATION

(11) **EP 4 201 392 A1**
(43) Date of publication of application: **28.06.2023**
(21) Application number: 21383174.6
(22) Date of filing: 21.12.2021
(51) Int. Cl.: A61K 8/02, A61K 8/73, A61Q 19/00

(54) **SOLID PERSONAL CARE PRODUCT AND METHOD TO OBTAIN SUCH PRODUCT**

(71) Applicant: Universitat Internacional De Catalunya, Fundació Privada, 08017 Barcelona (ES)
(72) Inventor: PÉREZ ANTOÑANZAS, Román, 08850 Gavà (ES); BOSCH CANALS, Begoña Maria, 08173 Sant Cugat del Vallès (ES)
(74) Representative: Juncosa Miró, Jaime

(57) **Abstract**

It is provided a personal care product adapted to provide a care function by being water-soluble comprising: a freeze-dried solid composition comprising a thickening agent, the composition adapted to provide a personal care function by being water-soluble; and a dried solid film comprising a thickening agent, the film adapted to provide a care function by being water-soluble; and wherein the dried solid film acts as a first enveloping element covering the freeze-dried solid composition. The personal care product is suitable for cosmetic, oral care, dermatological and pharmaceutical use. Said product can further comprise a second enveloping element comprising a synthetic polymeric film and on the inner part a thickening agent. Furthermore, the present invention is directed to a method to prepare said product.

## Description

### TECHNICAL FIELD

The present invention relates to solid products useful for personal care, i.e., a freeze-dried solid composition, a dried solid film and an enveloping element, which are soluble and at the same time sustainable. It further relates to a method to obtain such products.

### BACKGROUND ART

Cosmetic products containing active ingredients are typically provided to consumers in the form of liquid or pastes, such as cleansers, hair gels or toothpastes, thus requiring some form of external packaging. However, waste packaging is a significant problem from an environmental perspective, despite the availability of recycling. Cosmetic containers are often made from plastic materials, and regardless of whether these containers are recycled or partially recycled, the use of packaging is inherently damaging the environment at least because of the energy and water required to manufacture and transport the packaging.

Nevertheless, today this problem has been exacerbated even more, as the need to transport personal care products to different destinations has increased in recent years. As a result, the demand for easy-to-use, customized, portable cosmetic products has increased, and packaging waste has risen accordingly.

On the other hand, safety of cosmetic products has always been of special consideration for industries as microbial spoilage or air contact can lead to product degradation, or in the case of pathogens, an intimate contact with broken or damaged skin can cause a hazard for the health of the consumer. In order to guarantee cosmetic safety, a combination of preservatives is usually added to cosmetic products.

Preservatives are chemicals added to cosmetics to protect them against microbial or environmental insults occurring from raw materials, manufacture and consumer use. However, many questions have arisen regarding the safety of chemical preservatives. The prevalence of cosmetic-related contact allergy or sensory irritation in general population is mostly linked to the presence of preservatives.

These reasons have led some consumers to have a negative perception of cosmetics. Therefore, the cosmetic industry is looking for alternatives to the use of plastic containers and preservatives without compromising the microbiological stability, safety and efficacy of their products.

In recent years, a considerable interest has been brought to the development of preservative-free or self-preserving cosmetics that are more tolerable to the consumers. Preservative-free formulations can be made microbiologically stable by sterile production and appropriate packaging, therefore most preservative-free aqueous cosmetics packaged in multi-use containers cannot maintain the required conditions once opened. Thus, the most used preservative-free products are aqueous formulations contained in single-dose containers. Nevertheless, although this can help to obtain preservative-free cosmetic products, this type of solution is not sustainable.

There have been several attempts to respond to such demands, through the provision of solid cosmetic products that avoid the need to use external packaging and that are redissolved in water at the time of use, thus making them preservative-free.

EP0412449B1 relates to a solid cosmetic composition comprising a freeze-dried product containing polyethylene glycols and a thickener or a humectant. This document specifies that the solid composition is preferably dissolved in a solvent such as deionized water or any mixture of deionized water with one or more of lower aliphatic alcohols (e.g., ethanol).

WO2018235147A1 and JP2017110001A relate to a freeze-dried cosmetic tablet comprising trehalose and polyethylene glycol, and can further comprise a thickener and hyaluronic acid.

EP1613277B1 relates to a solid cosmetic composition comprising a water dissolvable solid carrier comprising a destructurized starch, at least one cosmetic agent and a fragrance. There is a step in the method of synthesis that implies the mixing of the composition at high temperatures of 150-250°C.

However, these solid compositions have some limitations in terms of use and synthesis. Some of them comprise polyethylene glycols, which are synthetic polymers that do not have the appropriate solubility in water to easily dissolve the solid composition. In addition, they are susceptible to oxidative degradation in the presence of air, which could jeopardize stability. Polyethylene glycols are also incompatible with some compounds (e.g., sorbitol, tannic acid or salicylic acid), so they may affect the integrity of plastics. On the other hand, some of the above-mentioned solid compositions need to be synthesized at high temperatures of up to 150-250 °C, thereby limiting the type of nature of the cosmetic active ingredients introduced into the product.

Therefore, there is a need in the art to provide alternative personal care products that are self-preserving and at the same time sustainable. Particularly there is a need to provide solid cosmetic compositions that are easily dissolvable in water and therefore simple to use, and that can be synthesized at low temperatures, so that multiple cosmetic active ingredients can be added therein, making it a versatile solid composition with different personal care usages.

### SUMMARY OF THE INVENTION

One problem to be solved by the present invention may be seen as related to the provision of a self-preservative personal care product capable of maintaining the safety and efficacy without compromising sustainability.

The solution is based on the provision of solid compositions, which are water and humidity free and are naturally preserved, that is, no preservative agents are included in said composition and the components are stable during storage, and which does not require the use of an ordinary external packaging.

The present invention relates to a solid composition, a dried solid film and an enveloping element, which can either be used as a personal care product and/or the latter two, as an external enveloping element.

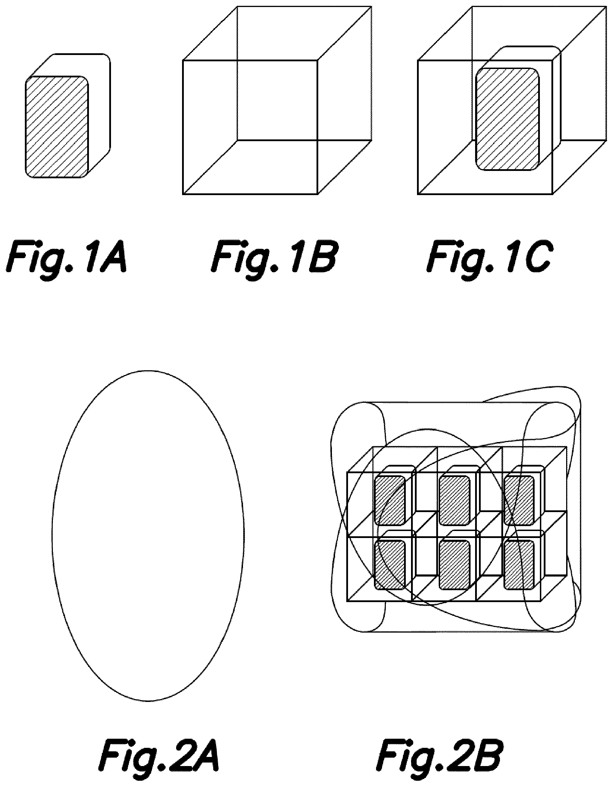

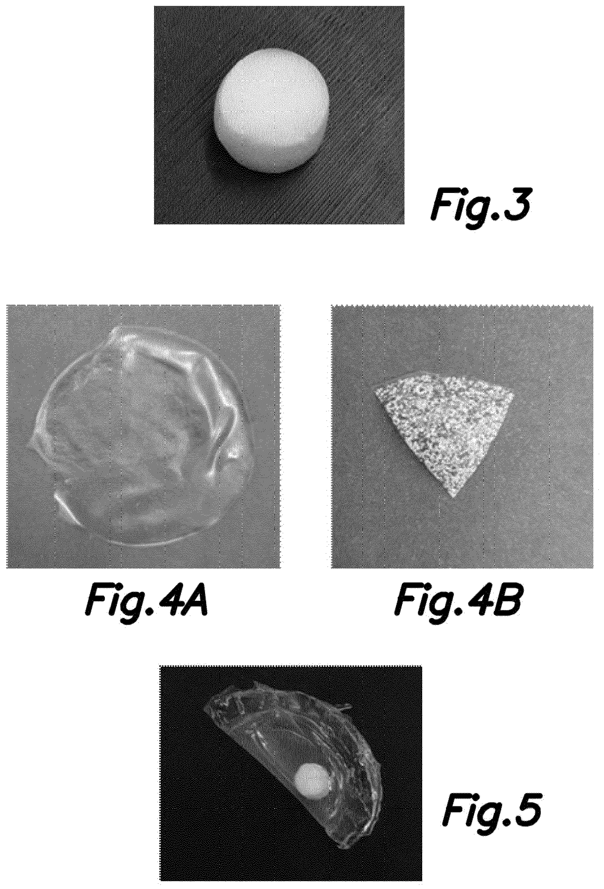

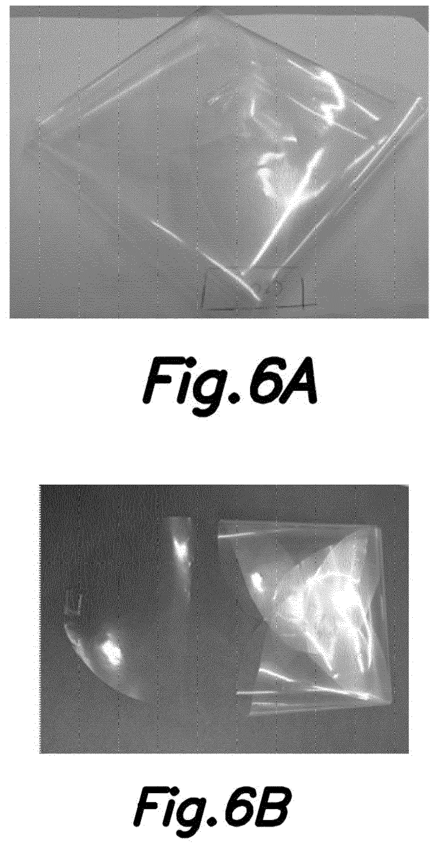

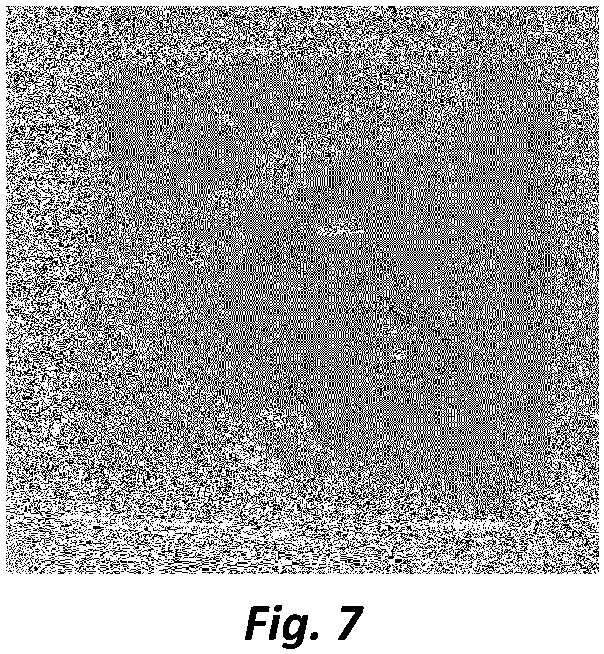

In particular, the present invention relates to a freeze-dried solid composition (e.g., Fig. 1A, Fig. 3), a dried solid film (e.g., Fig. 1B, Fig. 4A-B) and an enveloping element (e.g., Fig. 2A, Fig. 6A-B), which can either be used as a personal care product and/or the latter two, as an external enveloping element. The freeze-dried solid composition which can further contain an active ingredient, can be covered by the dried solid film (e.g., Fig. 1C, Fig. 5), thereby the latter acting as a first enveloping element. If acting as a first enveloping element, the dried solid film prevents the freeze-dried solid composition from degradation caused by microbial or environmental insults. The invention can further comprise a second enveloping element which can be used as an external packaging and also for personal care (e.g., Fig. 2B, Fig. 7).

Further, and even though it can be single-use, the invention is sustainable as the packages are completely or partially consumed, since every part of the product has a personal care function. In addition, all components are biodegradable so that in the case that the product is partially consumed, it does not alter its sustainability.

Apart from the safety that solid compositions provide, the efficacy of their active ingredients is maintained and even improved, since the activity and stability of the active ingredient is greater than that of the active ingredient by itself. An additional advantage of the solid compositions is that can include high amounts by weight of active ingredient, such as vitamin C. Moreover, a characteristic of all basic components of the solid compositions of the present invention, in addition to those of the active ingredients, is their non-toxic, non-irritant and skin moisturizing properties, thus acquiring a personal care function *per se.* Some solid compositions known in the art do not meet this characteristic (e.g., compositions comprising polyethylene glycol at high doses may be toxic).

Moreover, the solid compositions of the invention can provide multiple personal care functions, e.g., as an exfoliant, moisturizer, cleanser or make-up remover, among others. Further, the freeze-dried solid composition can even be made up of different layers, each of which providing different personal care functions. For example, the freeze-dried solid composition can be made of some basic components throughout different layers (e.g., alginate, cellulose and glycerin), having different components at each layer (e.g., cleansing and moisturizing active ingredients separately located) to be applied consecutively.

Further, the present invention also relates to methods of preparation of such personal care products. It is noted that the solid compositions can be prepared and applied to the body at low temperatures without compromising any of the active ingredients that can be added therein. Thus, the inventors provide solid compositions that are highly versatile, due to the wide variety of components and personal care uses that can have accordingly.

Accordingly, a first aspect of the invention relates to a personal care product adapted to provide a care function by being water-soluble, comprising: a solid composition adapted to provide a personal care by being water soluble, and a dried solid composition adapted to provide a personal care function by being water-soluble; and a dried solid film adapted to provide a care function by being at least partially water-soluble, wherein the dried solid film acts as a first enveloping element covering the freeze-dried composition.

A second aspect relates to a personal care product adapted to provide a care function by being water-soluble, comprising: a freeze-dried solid composition (also referred herein as "base") comprising at least one thickening agent, the composition adapted to provide a personal care function by being water-soluble; and a dried solid film comprising at least one thickening agent, the film adapted to provide a care function by being water-soluble, wherein the dried solid film acts as a first enveloping element covering the freeze-dried solid composition (e.g., Fig. 5).

A third aspect relates to a method to prepare a personal care product comprising the steps of: (a) preparing a freeze-dried solid composition comprising the steps of: (i) preparing a solution of at least one thickening agent and water; (ii) stirring the solution until its homogenization; (iii) freezing the solution; and (iv) freeze-drying the frozen solution; (b) preparing a dried solid film comprising the steps of: (i) preparing a solution of at least one thickening agent and water; (ii) placing the solution on a mold; and (iii) drying the solution to form a dried film; (c) covering the freeze-dried solid solution with the dried solid film; and (d) sealing the edges of the dried solid film by applying temperature, pressure and/or a salt solution.

A fourth aspect relates to a personal care product obtainable by the method as defined in the second aspect of the present invention.

A fifth aspect relates to a freeze-dried solid composition comprising at least one thickening agent, the composition adapted to provide a personal care function by being water-soluble (e.g., Fig. 3).

A sixth aspect relates to a dried solid film comprising at least one thickening agent, the film adapted to provide a personal care function by being water-soluble (e.g., Fig. 4A-B).

A seventh aspect relates to an enveloping element for use as external packaging comprising a biodegradable and synthetic polymeric film with an inner coating made of at least one thickening agent, the inner coating being adapted to provide a personal care function by being water-soluble (e.g., Fig. 6A-B).

An eighth aspect relates to a method to prepare a freeze-dried solid composition comprising the steps of: (i) preparing a solution of at least one thickening agent and water; (ii) stirring the solution until its homogenization; (iii) freezing the solution; and (iv) freeze-drying the frozen solution.

A ninth aspect relates to a freeze-dried solid composition obtainable by the method as defined in the seventh aspect of the present invention.

A tenth aspect relates to a method to prepare a dried solid film comprising the steps of: (i) preparing a solution of at least one thickening agent and water; (ii) placing the solution on a mold; and (iii) drying the solution to form a dried film.

An eleventh aspect relates to a dried solid film obtainable by the method as defined in the ninth aspect of the present invention.

A twelfth aspect relates to a method to prepare a personal care product comprising the steps of: (i) covering the freeze-dried solid composition with the first enveloping element; and (ii) sealing the edges of the first enveloping element by applying temperature, a salt solution and/or pressure.

A thirteenth aspect relates to a method to prepare an enveloping element comprising the steps of: (i) dissolving a synthetic polymer in an organic solvent or heating it at high temperature; (ii) pouring the resulting solution onto a mold; (iii) drying the solution to form a solid film; (iv) scratching one side of the film; (v) placing the at least one thickening agent on the scratched side; and (vi) drying the at least one thickening agent.

A fourteenth aspect relates to an enveloping element obtainable by the method as defined in the twelfth aspect of the invention.

Other aspects of the invention relate to a freeze-dried solid composition comprising at least one thickening agent, a dried solid film comprising at least one thickening agent, an enveloping element comprising a biodegradable and synthetic polymeric film with an inner coating made of a thickening agent, and combinations thereof.

Terms used herein are understood in its widely and common meaning in this description. Nevertheless, some are defined hereinafter in the detailed description of the invention.

### BRIEF DESCRIPTION OF THE DRAWINGS

Fig. 1A shows a schematic representation of the freeze-dried solid composition (base) according to an embodiment of present invention while Fig. 1B shows a schematic representation of the dried solid film according to an embodiment of the present invention. Fig. 1C shows a schematic representation of the dried solid film acting as a first enveloping element wrapped around the base.
Fig. 2A shows a schematic representation of the enveloping element according to an embodiment of the invention. Fig. 2B shows a schematic representation of the second enveloping element, in this case comprising two of the units depicted in Fig. 2A containing six first enveloping elements containing, each of them, one base according to an embodiment of the present invention.
Fig. 3 is a picture of the base according to an embodiment of the invention.
Fig. 4A is a picture of the dried solid film according to an embodiment of the invention. Fig. 4B is a picture of the dried solid film comprising exfoliating particles in one of the faces of the first enveloping element according to an embodiment of the invention.
Fig. 5 is a picture of the first enveloping element wrapped around the base according to an embodiment of the invention.
Fig. 6A and Fig. 6B are pictures of the enveloping element according to an embodiment of the invention. Fig. 6A shows a closed enveloping element, whereas Fig. 6B shows the enveloping element in an open configuration.
Fig. 7 is a picture of the second enveloping element wrapping the first enveloping element, which at the same time the latter wraps the base.

### DETAILED DESCRIPTION OF THE INVENTION

As described herein, in one aspect of the present invention, it is provided a freeze-dried solid composition comprising a thickening agent (e.g., alginate), the composition adapted to provide a care function by being water-soluble. This composition can further comprise an active ingredient (e.g., a vitamin) that can have an additional personal care function. The freeze-dried solid composition can also be referred herein as base, sponge, carrier or scaffold (e.g., Fig. 1A, Fig. 3).

In another aspect of the present invention, it is provided a dried solid film which comprises a thickening agent, wherein the film is adapted to provide a care function by being water-soluble. It can further comprise an active ingredient that can have an additional personal care function (e.g., Fig. 1B, Fig. 4A-B). Additionally, the dried solid film can also act as a first enveloping element, by covering the freeze-dried solid composition (e.g., Fig. 1C, Fig. 5).

In yet another aspect of the present invention, it is provided an enveloping element wherein its inner part comprising a thickening agent (i.e., inner coating) is adapted to provide a care function by being water-soluble and its outer part is adapted to be used as an external packaging by being water insoluble. This external packaging is biodegradable and flexible, which allows it to be shaped to a specific form, e.g., to be adapted to the shape of some part of the body (e.g., Fig. 2A, Fig. 6A-B). Said enveloping element can act as a second enveloping element, by covering the first enveloping element comprising the base (e.g., Fig. 2B, Fig. 7).

The term "packaging", in the context of the present invention, refers to any object, container or material used for containing any product or substance inside in connection with the packaging, transport, handling, protection, promotion, marketing or sale of said product or substance.

The different objects provided herein (i.e., the freeze-dried solid composition, the dried solid film and the enveloping element) are referred herein as "solid compositions", and can either be used individually or in combination.

Said solid compositions comprising a water-soluble thickening agent can release e.g., cosmetic or pharmaceutical material that the user may apply to their body (e.g., their skin, hair and/or teeth) upon contact of the solid composition with an aqueous solvent that is soluble in water. Upon contact with e.g., water, the cosmetic or pharmaceutical material may be released (e.g., at the surface of the skin) in the form of a non-solid (e.g., semi- solid or liquid) material. For example, the cosmetic material may be released at the surface of the skin in the form of a semi-solid (e.g., gel/paste) or liquid phase. When the solid composition is rehydrated and released, it can have an action on e.g., the skin through the constituent components that it contains (e.g., thickening agent or active ingredients), an action which can be carried out either by diffusion inside the skin (i.e., by transdermal skin) or by simple contact effect on the surface of the skin.

The solid compositions can be dissolved directly with an aqueous solvent on the user's body, or it can be dissolved previously and subsequently applied by the user on their body as desired. The dissolution can be enhanced by applying a relative movement to it with respect to the skin, having a massaging effect, so as to spread said rehydrated composition on the surface of the skin (i.e., over a larger area than the surface of the solid compositions) and facilitate its possible penetration.

In a particular embodiment, the personal care function is provided upon contact and/or friction with an aqueous solvent, particularly water. In an embodiment, the personal care function is provided upon contact and/or friction with an aqueous solvent at a temperature below 40 °C, particularly at room temperature. All the components of the solid compositions are soluble in water at low temperatures, therefore it is not necessary to rehydrate the solid compositions at high temperatures, which could jeopardize the active ingredients. Working at low temperatures allows better preservation of the active ingredients present in the solid compositions.

According to one embodiment, the solid compositions are capable of dissolving rapidly in an aqueous solvent. According to one embodiment, the dissolution time in water is between 0 minutes and 5 minutes, particularly between 0 seconds and 60 seconds.

It is particularly noted that, even after contacting the solid compositions with water, the compositions are able to dry and be absorbed after a relatively short period of time, so as to provide a non-tacky and dry solid composition. Thus, the personal care action can be obtained rapidly, in particular in a few minutes, or result from a longer contact, for example one hour.

For ease of reference, these and further aspects of the present invention are now discussed under appropriate section headings. However, the teachings under each section are not necessarily limited to each particular section.

### Thickening agent

As described herein, the solid compositions comprise at least one thickening agent. The thickening agent can be a hydrocolloid. As the skilled person will appreciate, hydrocolloids are a heterogeneous group of long chain polymers characterized by their property of forming viscous dispersions or gels when dispersed in water. Some hydrocolloids have the ability to modify the rheology of compositions comprising water. There are two types of hydrocolloids: gelling agents (i.e., those that modify the texture and/or solidity of the composition) and thickening agents (i.e., those that modify the viscosity of the composition). The water-thickening property is common to all hydrocolloids, and thus gelling agents can be regarded as a subset of thickening agents. For this reason, the terms "thickening agent", "thickener", "gelling agent", "gelifier agent", "gelifier" and "rheology modifier" can be used interchangeably.

Particularly, the solid compositions that comprise a thickening agent are the freeze-dried solid composition, the dried solid film and the internal coating of the second enveloping element. Because the thickening agent is water-soluble, these compositions have the ability to dissolve upon contact with an aqueous solvent that is soluble in water.

The compounds used as thickeners are well known in the art, so the amounts of agents will be determined by the skilled in the art. The thickening agent can be, but is not limited to, polysaccharides, plant-base extracts, polypeptides, polymers, monosaccharides or glycoproteins.

In an embodiment, the thickening agent is a polysaccharide. "Polysaccharides" refer to gelling agents that contain a backbone of repeating sugar (i.e., carbohydrate) units. The polysaccharide can be, but is not limited to, gums, natural polysaccharides, cellulose, cellulose derivatives or starches. In some embodiments, the polysaccharide is selected from the group consisting of a gum, a natural polysaccharide, a cellulose, a cellulose derivative and a starch.

In a particular embodiment, the thickening agent is a gum. The gum can be, e.g., xanthan gum, Arabic gum, guar gum, tragacanth gum, gum karaya, locust bean gum, carob bean gum, acacia gum, ghatti gum, gellant gum, karaya gum, konjac gum, hakea gum and tara gum. Such gums can be generally classified as carbohydrate gums that have an overall negative charge. In a more particular embodiment, the gum can be xanthan gum or gellant gum. In a more particular embodiment, the gum is xanthan gum. In another particular embodiment, the gum is gellant gum.

Other thickening and gelling agents useful herein include materials which are primarily derived from natural sources. In a particular embodiment, the thickening agent is a natural polysaccharide. Non-limiting examples of these gelling agents can be acacia, agar, algin, alginic acid and its salts (alginates), ammonium alginate, calcium alginate, sodium alginate, potassium alginate, propylene glycol alginate, carrageenan, calcium carrageenan, potassium carrageenan, sodium carrageenan, amylopectin, carnitine, dextrin, gellant gum, guar gum, guar hydroxypropyltrimonium chloride, hectorite, hyaluronic acid, sodium hyaluronate, hydrated silica, chitosan, hydroxypropyl chitosan, hydroxypropyl guar, karaya gum, kelp, locust bean gum, natto gum, sclerotium gum, sodium carboxymethyl dextran, tragacanth gum, xanthan gum, and mixtures thereof. In a particular embodiment, the thickening agent can be alginate, sodium hyaluronate or chitosan. In a more particular embodiment, the thickening agent is alginate. In another particular embodiment, the thickening agent is sodium hyaluronate. In another particular embodiment, the thickening agent is chitosan.

In a particular embodiment, the thickening agent is cellulose. In another particular embodiment, the thickening agent is a cellulose derivative. The main derivatives are cellulose ethers, i.e., alkyl modifications of cellulose, resulting from substituting part of the hydrogen atoms of the hydroxyl groups of the anhydrous glucose units with alkyl groups. Non-limiting examples of cellulose ethers are: methylcellulose (MC), hydroxyethylmethylcellulose (HEMC), hydroxypropylcellulose (HPC), hydroxypropylmethylcellulose (HPMC), hydroxyethylcellulose (HEC), ethylhydroxyethylcellulose (EHEC), ethylcellulose, sodium carboxymethylcellulose (CMCNa), quaternary ammonium salts of hydroxyethylcellulose with a trimethylammonium substituent (Polyquaternium 10), and dimethyldiallylammonium chloride copolymers (Polyquaternium A).

In a particular embodiment, the thickening agent is a starch. Non-limiting examples of starches include pregelatinized starch (e.g., corn, wheat, tapioca), pregelatinized high amylose content starch, pregelatinized hydrolyzed starches (e.g., maltodextrins, corn syrup solids), or chemically modified starches such as pregelatinized substituted starches (e.g., octenyl succinate modified starches). In a particular embodiment, the starch is pectin or maltodextrin. Further polysaccharides include starch derivatives (e.g., starch oxide, dialdehyde starch, dextrin, British gum, acetyl starch, starch phosphate, carboxymethyl starch, hydroxyethyl starch, hydroxypropyl starch).

In an embodiment, the thickening agent is a plant-based extract or vegetable extract. Non-limiting examples of vegetable extracts include psyllium gums, nuts, dates, arrowroots, coconut flour, chickpea flour, agar-agar, seeds (e.g., chia and flax), xanthan gum, oats, beans, root vegetables, potatoes, rice, coconut milk, tapioca starch, cornstarch, wheat flour or pectins. In a particular embodiment, the plant-based extract is a psyllium gum. Psyllium is a water-soluble gel-forming mucilage (also known as psyllium hydrocolloid or psyllium seed gum) obtained from the seed husk of *Plantago* genre plants, e.g., *Plantago ovate* or *Plantago psyllium.* In a particular embodiment, the thickening agent is psyllium, and more particularly *Plantago psyllium* seed extract.

In an embodiment, the thickening agent is a polypeptide. In a particular embodiment, the polypeptide is selected from the group consisting of gelatin, collagen, casein and silk fibroin.

In a more particular embodiment, the thickening agent is collagen. Silk fibroin may provide a moister feeling effect. In another particular embodiment, the thickening agent is silk fibroin.

In an embodiment, the thickening agent is a polymer. The polymer can be an acrylate-based polymer, a vinyl-based polymer (e.g., polyvinyl alcohol (PVA), polyvinylpyrrolidone) and polyalkylene glycol (e.g., polyethylene glycol). In a particular embodiment, the thickening agent is polyvinyl alcohol.

In an embodiment, the thickening agent is a monosaccharide, such as rhamnose, glucose, galactofuranose, galactose, mannose, allose, gulose, idose, talose, ribose, arabinose, xylose, erythrose, fucose, quinovose, and mixtures thereof. In a particular embodiment, the monosaccharide is rhamnose.

In an embodiment, the thickening agent is a glycoprotein, such as proteoglycans (e.g., heparan, chondroitin, keratan sulfate) or sericin.

In an embodiment, the composition of the base and the first enveloping element comprise natural compounds, in particular polysaccharides and/or plant extracts.

In a particular embodiment, the thickening agent is selected from the group consisting of alginate, cellulose, gellant gum, xanthan gum, rhamnose, silk fibroin, sericin, chitosan, carrageenan, dextrin, hyaluronic acid, sodium hyaluronate, hydroxypropyl chitosan, sodium alginate, potassium alginate, propylene glycol alginate, starch, pectin, maltodextrin, methylcellulose, hydroxypropylmethylcellulose, sodium carboxymethylcellulose, polyvinyl alcohol, psyllium and combinations thereof.

In a more particular embodiment, the composition of the thickening agent is selected from the group consisting of alginate, cellulose, gellant gum, xanthan gum, rhamnose, silk fibroin, sodium hyaluronate, chitosan, psyllium (particularly *Plantago psyllium* seed extract), polyvinyl alcohol and combinations thereof.

In Examples 1 and 3, different compositions comprising different thickening agents were successfully produced. The thickening agents tested were alginate, cellulose, gellant gum, xanthan gum, rhamnose, silk fibroin, sodium hyaluronate, chitosan, psyllium and polyvinyl alcohol (Tables 1 and 6). These compounds are a representation of other thickening agents listed herein, thus it is plausible that other compositions comprising thickening agents other than those tested will work, due to the similarity of their properties.

### Humectants, inorganic salts and pH adjusters

The solid compositions can further comprise other components selected from humectants (such as glycerin or urea), inorganic salts (such as sodium chloride), or pH adjusters (such as sodium hydroxide).

In an embodiment, the freeze-dried solid composition and/or the dried solid film further comprise at least one component selected from the group consisting of a humectant, an inorganic salt, and a pH adjuster.

In some embodiments, the solid compositions further comprise at least one humectant. It was found by the present inventors that the inclusion of a humectant not only improves the moisturizing properties of the product, but may also improves the ease of release of the material from the solid composition upon contact with water.

In some embodiments, the solid compositions further comprise a humectant selected from the group consisting of glycerin/glycerol, urea, propylene glycol, 1,3-propanediol, honey (or honey wax), sorbitol, glucose syrup, polysaccharide syrup, monosaccharide syrup, and mixtures thereof. In some embodiments, the humectant is selected from the group consisting of glycerin/glycerol, propylene glycol, 1,3-propanediol, honey, sorbitol, and mixtures thereof. In some embodiments, the humectant is selected from the group consisting of glycerin/glycerol, urea, propylene glycol, and mixtures thereof. In an embodiment, the humectant is glycerin/glycerol. In another embodiment, the humectant is urea. For instance, urea can provide a freshening effect, so it can be used for dry skins.

In some embodiments, the solid compositions further comprise at least one inorganic salt, such as, but not limited to, sodium chloride, calcium chloride, magnesium chloride, sodium bicarbonate, potassium chloride, sodium sulfate, calcium carbonate, calcium phosphate, and mixtures thereof. In a particular embodiment, the solid compositions further comprise a monovalent salt. In another particular embodiment, the solid compositions further comprise a divalent salt. Particularly, the solid compositions further comprise sodium chloride.

Inorganic salts can provide different functions, e.g., break more easily the alginate chains and thus increase the solubility of the base (e.g., sodium chloride or sodium sulfate); make bonds between alginate fibers to allow alginate gelation (e.g., calcium chloride, magnesium chloride, potassium chloride or calcium phosphate); have the benefit of calcium as a linker (e.g., calcium carbonate); or create bubbles upon dissolution (e.g., sodium bicarbonate or calcium carbonate). In addition, inorganic salts can have therapeutic or pharmacological effects, e.g., cobalt chloride or copper chloride eventually enhance angiogenesis. Inorganic salts can have a positive effect on healing, e.g., ions such as silver (Ag), copper (Cu), cobalt (Co), magnesium (Mg), silicon (Si) with their respective salts e.g., chloride, sulphate, phosphate, among others.

In some embodiments, the solid compositions further comprise at least one pH adjuster, such as, but not limited to hydroxides (e.g., sodium hydroxide or potassium hydroxide). In a particular embodiment, the solid compositions further comprise sodium hydroxide. pH adjusters can be added to the solid compositions in a suitable amount to achieve the desired pH. They can also act as moisturizers.

In Examples 1 and 3, different compositions comprising different humectants, inorganic salts and pH adjusters were successfully produced. The humectants tested were glycerin/glycerol, and urea, the inorganic salt tested was sodium chloride and the pH adjuster used was sodium hydroxide (Tables 1 and 6). These compounds are a representation of other humectants, inorganic salts and pH adjusters listed herein, thus it is plausible that other compositions comprising humectants, inorganic salts and pH adjusters other than those tested, will work, due to the similarity of their properties.

### Active ingredients

In addition to the above, the solid compositions can further comprise at least one active ingredient. As used herein, the term "active ingredient" comprises ingredients with an intended effect as e.g., a cosmetic or a pharmaceutical effect. The active ingredient can be an agent that renders the composition suitable for use in cosmetics or personal care, as e.g., a cleanser (cleaning the hair and/or body of a user), a moisturizer or a hand/body lotion, an exfoliant, a colour cosmetic (e.g., make-up, such as foundation, eye shadow, lipstick, blush or the like), an oral care product, a hair gelling product, a hair dye or hair colourant, among others.

In some embodiments, the active ingredient is a water-soluble and/or liposoluble agent. In some embodiments, the active ingredient is a water-soluble agent, e.g., a water-soluble vitamin such as vitamins B1, B2, B3, B5, C, B6, B7, B9 or B12, and/or a liposoluble vitamin, such as vitamins A, D, E or K. In some embodiments, the liposoluble agent is e.g., lecithin, lanolin, rosehip wax, squalene, beeswax, plant waxes, farnesol, gama oryzanol, zinc ricinoleat, stearic acid, vegetable cetyl alcohol, cetostearyl alcohol, linoleic acid, or palmitic acid. In some embodiments, the water-soluble agent is e.g., alpha hydroxy acids (AHAs) such as citric acid, lactic acid, malic acid, sodium coco sulfate, glycerin, zinc pyrrolidone carboxylic acid (PCA), hydrolyzed wheat protein, sodium cocoyl glutamate, or sodium lauroyl glutamate.

In some embodiments, the active ingredient is a perfume, fragrance or aroma (essential oil). Fragrances can be added to the product to make the experience of using the present composition more pleasant. Essential oils can be selected based on the fragrance desired, skin type to be treated and other effects desired based on the well-known properties of essential oils. Many well documented effects can be achieved by the use of essential oils. In one embodiment, the one or more essential oils present in the solid compositions can be, but are not limited to, aloe vera, menthol, tarragon, lemon myrtle, jasmin, ylang ylang, labdanum, lemongrass, rose otto, grapefruit, patchouli, rosemary, armois, lemon, neroli, sweet violet, lavender, orange, vanilla, peppermint, benzoin, hydrangia, litsea cubeba, cardamon, tonka, and chamomile blue. In a particular embodiment, the aroma can be selected from the group consisting of aloe vera, menthol, lavender and vanilla.

In some embodiments, the active ingredient is a vitamin and/or vitamin derivatives. Vitamins, particularly A, B, C and E are very beneficial for the skin. Vitamin rich ingredients such as wheatgerm oil can also be used to deliver vitamins on to the skin. In an embodiment, vitamins and vitamin derivatives are selected from retinoic acid (derived from vitamin A), vitamin A, vitamin B, vitamin C, vitamin E and mixtures thereof. It will be appreciated by one skilled in the art that vitamin can be provided from any suitable source. For example, vitamin(s) can be provided from a synthetic source or from incorporation into the product of a material, such as a natural material, that has a high vitamin content. In a particular embodiment, vitamin is vitamin A, C and E. In a particular embodiment, vitamin is vitamin B3.

In some embodiments, the active ingredient can have e.g., a slimming, cell renewal, anti-inflammatory, anti-wrinkle, keratolytic, anti-sebum, brightening effect, among others.

In some embodiments, the active ingredient is selected from the group consisting of anti-stress agents, detox/purifying or slimming agents, moisturizing agents, regenerative or cell renewal agents, firmness/lifting effect agents, anti-wrinkle/anti-aging agents, flash/brightness agents, anti-acne or anti-sebum agents, freshness effect agents, heat effect agents, soft effect agents, anti-irritation agents/painkillers or healing agents, anti-blemish agents, dry-skin agents, anti-inflammatory agents, anti-spot agents, cleansing agents, exfoliant agents, UV absorbing materials/sun filters, UV reflecting materials/sunscreens, bronzer agents and, anti-aggression agents.

In an embodiment, the active ingredient is an anti-stress agent (e.g., base of *Tephrosia purpurea*).

In an embodiment, the active ingredient is a detox/purifying or slimming agent (e.g., active carbon, micro-algae extract, grape oil, *Centella asiatica* extract, *Garcinia* extract, *Coleus* extract (containing forskohlina) or caffeine).

In an embodiment, the active ingredient is a moisturizing agent (e.g., aloe vera, urea, hyaluronic acid, silk, rosehip oil, coconut oil, olive oil, *Calendula* extract, collagen, ceramides, squalene, or peroxidized triglycerides).

In an embodiment, the active ingredient is a regenerative or cell renewal agent (e.g., *Centella asiatica* extract, *Calendula* extract, aloe vera, silk, royal jelly, rosehip oil, red algae extract (*Chondrus crispus*), or *Mimosa tenuiflora* extract).

In an embodiment, the active ingredient is a firmness/lifting effect agent (e.g., hyaluronic acid, silk, or collagen).

In an embodiment, the active ingredient is an anti-wrinkle/anti-aging agent (e.g., ursolic acid, hyaluronic acid, alpha hydroxy acid, silk (antioxidant), squalene, coconut oil, olive oil (it has vitamin E), or organic silicon).

In an embodiment, the active ingredient is a flash/brightness agent (e.g., collagen, carnauba wax, azelaic acid or kojic acid).

In an embodiment, the active ingredient is an anti-acne (antibacterial) or antisebum agent (e.g., *Calendula* extract, aloe vera, coconut oil, benzoyl peroxide, sulfur, *Mimosa tenuiflora* extract, azelaic acid, salicylic acid or hydroxy decanoic acid).

In an embodiment, the active ingredient is a freshness effect agent (e.g., aloe vera, or menthol) or a heat effect agent (e.g., *Capsicum* extract).

In an embodiment, the active ingredient is a soft effect agent (e.g., *Calendula* extract, hyaluronic acid, *Mimosa tenuiflora* extract, squalene or chamomile).

In an embodiment, the active ingredient is an anti-irritation agent/painkiller (e.g., aloe vera, chamomile, *Calendula* extract, or menthol) or a healing agent (such as *Mimosa tenuiflora* extract).

In an embodiment, the active ingredient is an anti-blemish agent (e.g., aloe vera, rosehip oil, coconut oil (anti-eye bags), or sea fern algae extract).

In an embodiment, the active ingredient is a dry-skin agent (e.g., shea butter, cetearyl olivate, squalene, sorbitan olivate, *Persea Gratissima* (avocado) oil, phytosterols, *Olea Europaea* (olive) fruit oil), mattifying agents (white claim such as kaolin), keratolytic agents (e.g., glycolic acid, lactic acid, salicylic acid, urea, alantoin, or trichloroacetic acid)).

In an embodiment, the active ingredient is an anti-inflammatory agent (e.g., glycyrrhetinic acid, niacinamide, *Andibora* seed oil, aloe vera, nordihydroguaiaretic acid, or ximenic acid).

In an embodiment, the active ingredient is an anti-spot agent such as bearberry extract (containing arbutin).

In some embodiments, the active ingredient is a cleansing agent (e.g., coconut oil, or *Mimosa tenuiflora* extract). Particularly, the active ingredient is a surfactant. The inclusion of a surfactant can render the product suitable for use in cleaning the hair, skin and/or teeth of the user. Therefore, in some embodiments, the solid compositions comprise a surfactant as an active agent, and is a solid shower gel, solid shampoo and/or solid oral care product (such as a solid toothpaste). In some embodiments, the surfactant is selected from the group consisting of anionic surfactant, amphoteric surfactant, cationic surfactant, non-ionic surfactant and mixtures thereof. In some embodiments, the surfactant is an anionic surfactant selected from the group consisting of sodium lauryl sulfate, sodium laureth sulfate, sodium dodecyl sulfate, sodium cocosulfate, ammonium lauryl sulfate, ammonium laureth sulfate, sodium myreth sulfate, sodium lauroyl sarcosinate, disodium laureth sulfosuccinate, disodium lauryl sulfosuccinate, sodium lauryl sulfoacetate, sodium cocoyl isethionate, sodium olefin sulfonate, and mixtures thereof. In some embodiments, the surfactant is an amphoteric surfactant selected from the group consisting of lauryl betaine, cocamidopropyl betaine, trimethyl glycine betaine, sodium cocoamphoacetate, disodium cocoamphodiacetate, sodium lauroamphoacetate, disodium lauroamphodiacetate, cocoamidopropyl hydroxysultaine, sodium lauroyl sarcosine, cetrimonium chloride and mixtures thereof. In some embodiments, the surfactant is selected from the group consisting of sodium lauryl sulfate, sodium laureth sulfate, sodium dodecyl sulfate, sodium cocosulfate, ammonium lauryl sulfate, ammonium laureth sulfate, sodium myreth sulfate, sodium lauroyl sarcosinate, disodium laureth sulfosuccinate, disodium lauryl sulfosuccinate, sodium lauryl sulfoacetate, sodium cocoyl isethionate, sodium olefin sulfonate, lauryl betaine, cocamidopropyl betaine, trimethyl glycine betaine, sodium cocoamphoacetate, disodium cocoamphodiacetate, sodium lauroamphoacetate, disodium lauroamphodiacetate, cocoamidopropyl hydroxysultaine, sodium lauroyl sarcosine, cetrimonium chloride and mixtures thereof.

In some embodiments, the active ingredient is an exfoliating material. This is an exfoliant abrasive component which facilitates removal of the substances and debris. It has been suggested that exfoliative cleaning promotes regeneration of the epidermal tissues such that the skin regains suppleness. It has also been proposed that the penetration of cosmetic or dermo-pharmaceutical products is facilitated by exfoliation. In some embodiments, the exfoliant materials include hydrogenated fats, inorganic salts such as sodium citrate or relatively low-molecular weight organics, such as sugars, synthetic polymers such as polyethylene powders and granulated particles (e.g., silica particles such as silicon dioxide) or organo-polysiloxane. The exfoliating material can be microparticles or microspheres, salts and/or sugars. For instance, exfoliant compositions can comprise e.g., polyethylene, microcrystalline wax, synthetic wax, jojoba esters, hydrogenated jojoba, amorphous silica, agglomerated silica, talc, tricalcium orthophosphate, or blends thereof.

In a particular embodiment, the exfoliating material comprises microspheres, particularly, silica microspheres. Besides providing an exfoliating effect, they can also help retaining moisture and avoid damaging the active ingredient. In addition, silica microspheres can further incorporate therein any other compound, e.g., an active ingredient.

Inventors developed novel microcarriers made of sol-gel-derived bioactive glasses for effectively delivering molecules, e.g., cosmetic or therapeutic molecules (such as proteins, growth factors, among others). Silica microspheres can be synthesized as follows.

The sol-gel process is a chemical method for producing solid materials using small molecules (monomers) as raw materials. These monomers are able to convert into a colloidal solution (sol) which is then transformed into an integrated network (gel). The sol is generally catalyzed into gel through the changes in different parameters such as pH or temperature.

Tetraethyl orthosilicate (TEOS, C₈H₂₀O₄Si, 98%) is mixed with 0.1 M HCI, with the addition of deionized water to form an acid catalyzed sol. The molar ratio of total water (includingCaCl₂ and HCI water) to TEOS is 8. The microspheres are doped with calcium ions by incorporating specific amounts of calcium chloride (CaCl₂) into the solution. The molar percentage of doped calcium ranged from 0 to 30. Once the sol was obtained, it is cooled down in a water bath at 4 °C. Afterwards, 0.08 M ammonium hydroxide (NH₄OH, 28.0% NH₃ in water, P99.99% metal basis) is added dropwise to the sol with agitation. The pH is adjusted to 5-5.5, and 5 ml of the sol is then added dropwise to 100 ml of olive oil, and is stirred at 95 rpm to allow gelation. Gelled microspheres are gathered after precipitation at the bottom of the flask, vacuum filtered, rinsed with water and ethanol, and left overnight to dry. To prepare a powder out of the silica, there is no need to prepare the emulsion. After incorporating the 0.08 M NH₄OH, the sol is left until gelation and afterwards ground with a mortar.

In an embodiment, the active ingredient is a UV absorbing material/sun filter (e.g., octocrylene, or butyl methoxydibenzoylmethane), a UV reflecting material/sunscreen (e.g., titanium dioxide, micronized titanium, micronized zinc oxide, or octyl methoxycinnamate), a bronzer agent (e.g., dihydroxy acetone, or erthrulose), or an anti-aggression (UV protection and pollution) agent (e.g., ectoin).

In some embodiments, the active ingredient is a protein. The protein can be selected from the group consisting of tofu, banana, soya, soya lecithin, hydrolyzed wheat protein, and mixtures thereof.

In some embodiments, the solid compositions comprise a fruit, fruit juice, and/or fruit extract. The fruit can be any suitable fruit. Particularly, the fruit can be selected from orange, mango, papaya, strawberry, banana, kiwi, apple, cherry, pineapple, raspberry, blueberry, blackberry, peach, nectarine, and mixtures thereof.

In some embodiments, the active ingredient is an oral care agent. For the avoidance of doubt, as used herein "oral care" is synonymous with oral hygiene. In such embodiments, the solid cosmetic composition is a solid oral care product, such as a solid toothpaste or solid mouthwash. In such embodiments, the solid cosmetic composition is thus suitable for use in cleaning or whitening the teeth of the user and/or freshening the breath of the user. The oral care agent can be selected from the group consisting of calcium carbonate, aluminum hydroxide, calcium hydrogen phosphate, silica, zeolite, sodium fluoride, stannous fluoride, olaflur, sodium monofluorophosphate, xylitol, sodium bicarbonate, chlorhexidine, cetylpyridinium chloride, hexetidine, triclosan, mentol, stevia, sucralose, fluorine and mixtures thereof.

In some embodiments, the active ingredient is a hair gelling agent, e.g., polyvinylpyrrolidone (PVP), PVP and dimethylaminoethylmethylacrylate copolymer, carbomer, vinylpyrrolidone (VP) and vinyl acetate (VA) copolymer, VP and dimethylaminopropylamine (DMAPA) acrylates copolymer, polyacrylate-2-crosspolymer, sodium polyitaconate, VP/methacrylamide/vinyl imidazole copolymer, PVP K-90, or mixtures thereof.

In some embodiments, the active ingredient is a hair colouring agent (or dye). The colouring agent can be naturally derived or it can be synthetic. Particularly, it is naturally occurring. A hair colouring agent is particularly any colouring that is completely or significantly soluble in an aqueous medium.

In some embodiments, the active ingredient is a pigment or skin colouring agent. In some embodiments, the pigment is an organic pigment, an inorganic pigment, or mixtures thereof. In some embodiments, the pigment is a natural pigment. The natural pigment can be selected from the group consisting of beetroot, chlorophyll, gardenia, blackberry, coffee, rose, caramel powder, grape, alfalfa, walnut hull, calendula, cocoa, green tea, hibiscus, kelp, olive, orange, parsley, pumpkin, spinach, spirulina, wheatgrass sources, turmeric, butterfly pea, carrot, tomato and mixtures thereof. In some embodiments, the pigment is an inorganic pigment selected from the group consisting of iron oxide, mica (e.g., synthetic mica), titanium dioxide, tin oxide, zinc oxide, and mixtures thereof.

In addition to the above, the solid compositions can further comprise one or more additives, e.g., cosmetically or pharmaceutically acceptable additives. In some embodiments, the one or more additives are selected from preservatives, wax, binders, fillers, clays, pacifiers, essential oils, sugar, bleach, vegetable butter, vegetable oil, honey, herb extracts, decorative items, emollients, effervescent components, and mixtures thereof. It is noted that preservatives could be added if needed in terms of regulatory requirements.

In a particular embodiment, the solid compositions further comprise sugar, so that they can be edible. In a particular embodiment, the freeze-dried solid composition further comprises a sugar and a vitamin. In a more particular embodiment, said composition is in the form of an edible sweet.

In a particular embodiment, the solid compositions further comprise a disinfectant such as bleach, so that they can be used for home care.

In an embodiment, the active ingredient is selected from the group consisting of aloe vera, menthol, geranium, lavender, urea, hyaluronic acid, silicon, organic silicon, silk, silica, vitamin A, vitamin B3, vitamin C, vitamin E, *Tephrosia purpurea, Mimosa tenuiflora, Capsicum, Calendula,* micro-algae extract, red algae extract (*Chondrus crispus*), sea fern algae extract, rosehip oil, coconut oil, olive oil, grape oil, cetearyl olivate, sorbitan olivate, *Persea Gratissima* (avocado) oil, phytosterols, *Olea Europaea* (olive) fruit oil, collagen, royal jelly, carnauba wax, active carbon, chamomile, shea butter, white claim (kaolin), and combinations thereof. In a particular embodiment, the active ingredient is selected from the group consisting of aloe vera, menthol, geranium, lavender, urea, hyaluronic acid, silicon, silk, silica, vitamin A, vitamin B3, vitamin C, vitamin E and combinations thereof.

In Example 2, different compositions comprising different active ingredients were successfully produced (Table 4), and their effects on the skin were tested (Table 5). In particular, the active ingredients tested were aloe vera, menthol, geranium, lavender, urea, hyaluronic acid, silicon, silk, vitamin A, vitamin B3, vitamin C and vitamin E (Table 3).

Results show that, depending on the active ingredient added in the solid composition, it is possible to obtain different personal care functions. In the working example, different personal care compositions were successfully prepared and shown to exhibit the appropriate effect according to the active ingredient added therein, such as moisturizers, refresheners, aromas, layered compositions with cleansing and moisturizing effects or with moisturizing effects for very dry skins, brighteners, firmness action compositions, anti-acne or anti-aging compositions.

Therefore, compositions comprising other active ingredients as e.g., the listed herein are to show an effect in accordance with the nature of the active ingredient, since the composition of the solid composition comprising a thickening agent (and optionally humectants and/or inorganic salts) does not interfere with the action of said active ingredient, but rather enhances it.

### Synthetic polymers

As described herein, the second enveloping element comprises a polymeric film with a thickening agent coating on the inner side. The polymer is insoluble in water, so that when it is mixed with water, it does not dissolve and remains intact. Nevertheless, the polymer can be both biodegradable and degradable, i.e., it can be degraded over long periods of time (i.e., slow degrading materials or polymers, which can be dissolved with organic solvents and/or melt at high temperatures). Thus, the second enveloping element is made up of a synthetic and degradable polymeric film, suitable for use as external packaging.

Non-limiting examples of these synthetic polymers include polyglutamic acid (PGA), poly(aspartic acid) (PASP), polylactic acid (PLA), poly(lactic-co-glycolic acid) (PLGA), poly caprolactone (PCL), polyhydroxyalkanoates (PHAs), poly(p-dioxanonse), polyurethane, nylon, polyphosphor ester, polyglycolic acid, or the pegylated forms of said polymers. Combinations of these and/or other polymers are also used in certain embodiments. In a particular embodiment, the second enveloping element comprises natural-based polymers. In a more particular embodiment, the second enveloping element comprises PLA and/or PCL.

### Compositions

The thickening agent and humectant components are the majority of components in the solid compositions, being the ones adapted to be dissolved and/or consumed and/or being used up. Other components, such as inorganic salts, pH adjusters or active ingredients, can be found in minority proportions, mainly to provide stability, pH regulation to the base and/or the first and/or the second enveloping elements.

The thickening agent can be included in an amount of from about 0.1% to about 100% by weight of the solid composition. In a particular embodiment, the thickening agent can be included in an amount of about 0.1%, 0.5%, 0.8%, 1.0%, 2.0%, 2.5%, 3.0%, 4.0%, 5.0%, 6.0%, 7.0%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, 99%, 99.5%, 99.9% or 100% by weight of the solid composition.

When present, the humectant can be included in an amount of from about 0.1% to about 50% by weight of the solid composition, such as from about 1% to about 30% by weight of the solid composition, such as from about 5% to about 20% by weight of the solid composition. In a particular embodiment, the humectant can be included in an amount of about 0.5%, 1.0%, 5.0%, 10%, 12%, 15%, 17%, 18%, 19%, 20%, 25%, 30%, 35% or 40% by weight of the solid composition.

When the composition further comprises an active ingredient, the selection of the components in this weight ratio allows for a sufficient amount of the active agent to be released from the product when the product is contacted with a small amount of water (e.g., when the user applies a small amount of water thereto).

The solid compositions provided herein have the advantage of being able to include high amounts by weight of active ingredient, such as vitamin C. Although the active ingredients can be added in low quantities, the high weights they can achieve result in a very high percentage by weight of the total solid composition (e.g., vitamin C can be included in an amount of about 75% by weight of the solid composition). This can be mainly observed with water-soluble active ingredients.

When present, the active ingredient can be included in an amount of from about 0.1 to about 75% by weight of the solid composition, particularly from about 0.1 to about 25% by weight of the solid composition. In a particular embodiment, the active ingredient can be included in an amount of about 0.1%, 0.5%, 0.8%, 1.0%, 2.0%, 2.5%, 3.0%, 4.0%, 5.0%, 6.0%, 7.0%, 10%, 11%, 12%, 13%, 14%, 15%, 16%, 17%, 18%, 19%, 20%, 25%, 50% or 75% by weight of the solid composition.

There are many compositions that are suitable for the solid compositions of the present invention. The following embodiments regarding each solid composition are not limiting examples of the compositions, but merely a representation thereof.

### Freeze-dried solid composition

The freeze-dried solid composition comprises at least one thickening agent as defined above. In a particular embodiment, the thickening agent is selected from the group consisting of natural polysaccharides or monosaccharides, polypeptides and combinations thereof.

Particularly, the thickening agent is a natural polysaccharide. In a particular embodiment, the natural polysaccharide is selected from the group consisting of alginate, cellulose, a gum, hyaluronic salts, and chitosan. Particularly, the gum is gellant gum or xanthan gum.

In an embodiment, the polypeptide is silk fibroin.

In an embodiment, the thickening agent is selected from the group consisting of alginate, cellulose, gellant gum, xanthan gum, rhamnose, silk fibroin, sericin, chitosan, carrageenan, dextrin, hyaluronic acid, sodium hyaluronate, hydroxypropyl chitosan, sodium alginate, potassium alginate, propylene glycol alginate, starch, pectin, maltodextrin, methylcellulose, hydroxypropylmethylcellulose, sodium carboxymethylcellulose, polyvinyl alcohol, psyllium and combinations thereof. In a particular embodiment, the thickening agent is selected from the group consisting of alginate, cellulose, gellant gum, xanthan gum, rhamnose, silk fibroin, proteoglycans, chitosan, hyaluronic acid, pectin and combinations thereof. In a particular embodiment, the thickening agent is selected from the group consisting of alginate, cellulose, gellant gum, xanthan gum, rhamnose, silk fibroin, sodium hyaluronate, chitosan and combinations thereof. In a particular embodiment, the thickening agent is alginate and/or cellulose, and more particularly alginate and cellulose.

In an embodiment, the freeze-dried solid composition comprising at least one thickening agent further comprises at least one humectant, pH adjuster and/or inorganic salt as defined above. In an embodiment, the freeze-dried solid composition comprising at least one thickening agent further comprises at least one humectant and at least one pH adjuster. In a particular embodiment, the humectant and/or pH adjusters are glycerin/glycerol, urea and/or sodium hydroxide.

Accordingly, the freeze-dried solid composition can comprise alginate, cellulose, gellant gum, xanthan gum, glycerin/glycerol, urea, rhamnose, silk fibroin, sodium hyaluronate, chitosan and/or sodium hydroxide. In a particular embodiment, the freeze-dried solid composition comprises alginate, cellulose and glycerin/glycerol.

Particularly, the freeze-dried solid composition can comprise alginate; or a combination of alginate and cellulose; or a combination of alginate, cellulose and glycerin/glycerol; or a combination of alginate, cellulose, glycerin/glycerol and urea; or a combination of alginate, cellulose, glycerin/glycerol, urea and xanthan gum; or a combination of alginate, cellulose, glycerin/glycerol, urea and gellant gum; or a combination of alginate, cellulose, glycerin/glycerol, urea, xanthan gum and gellant gum; or a combination of alginate, cellulose, glycerin/glycerol, urea, xanthan gum, gellant gum, and silk fibroin; or a combination of alginate, cellulose, glycerin/glycerol, urea, xanthan gum, gellant gum, silk fibroin, and sodium hyaluronate; or a combination of alginate, cellulose, glycerin/glycerol, urea, xanthan gum, gellant gum, silk fibroin, sodium hyaluronate, and rhamnose; or a combination of alginate, cellulose, glycerin/glycerol, urea, xanthan gum, gellant gum, silk fibroin, sodium hyaluronate, rhamnose, and chitosan. Sodium hydroxide is added in suitable amounts to said compositions to adjust pH.

The present invention also provides a freeze-dried solid composition as comprising 100% alginate by weight with respect to the total composition; or a combination of 30-40% alginate and 60-70% cellulose by weight with respect to the total composition; or a combination of 30-80% alginate, 15-60% cellulose and 1-10% glycerin by weight with respect to the total composition; or a combination of 30-80% alginate, 15-60% cellulose and 1-10% glycerin/glycerol by weight with respect to the total composition; or a combination of 15-25% alginate, 20-45% cellulose, 25-35% glycerin/glycerol, 3-25% urea by weight with respect to the total composition; or a combination of 10-50% alginate, 15-35% cellulose, 20-30% glycerin/glycerol, 2-35% urea and 0.2-4% xanthan gum by weight with respect to the total composition; or a combination of 10-50% alginate, 15-35% cellulose, 20-35% glycerin/glycerol, 3-35% urea and 0.2-4.5% gellant gum by weight with respect to the total composition; or a combination of 10-50% alginate, 15-35% cellulose, 15-35% glycerin/glycerol, 3-35% urea, 0.2-4.5% xanthan gum and 0.2-4.5% gellant gum by weight with respect to the total composition; or a combination of 10-50% alginate, 15-35% cellulose, 15-35% glycerin/glycerol, 3-35% urea, 0.2-4.5% xanthan gum, 0.2-4.5% gellant gum, 5-15% silk fibroin and 1-10% sodium hyaluronate by weight with respect to the total composition; or a combination of 10-50% alginate, 15-35% cellulose, 15-35% glycerin/glycerol, 3-35% urea, 0.2-4.5% xanthan gum, 0.2-4.5% gellant gum, 5-15% silk fibroin, 1-10% sodium hyaluronate and 5-15% rhamnose by weight with respect to the total composition; or a combination of 7-10% alginate, 10-20% cellulose, 2-3% gellant gum, 0.1-2.5% xanthan gum, 10-15% glycerin/glycerol, 1-12% urea, 10-12% silk fibroin, 1-5% sodium hyaluronate, 7-13% chitosan and 10-13% rhamnose by weight with respect to the total composition. Sodium hydroxide is added in suitable amounts to said compositions to adjust pH, particularly between 15-22% by weight with respect to the total composition, thereby altering the amounts by weight of the other components.

In a particular embodiment, alginate is in an amount of between 10-80% by weight with respect to the total composition, particularly between 15-75%. In a particular embodiment, cellulose is in an amount of between 10-60% by weight with respect to the total composition, particularly between 15-48%. In a particular embodiment, gellant gum is in an amount of between 0.1-5% by weight with respect to the total composition, particularly between 0.8-2.5%. In a particular embodiment, xanthan gum is in an amount of between 0.1-5% by weight with respect to the total composition, particularly between 0.2-1%. In a particular embodiment, glycerin/glycerol is in an amount of between 1-35% by weight with respect to the total composition, particularly between 5-30%. In a particular embodiment, urea is in an amount of between 0.1-35% by weight with respect to the total composition, particularly between 1-30%. In a particular embodiment, rhamnose is in an amount of between 3-20% by weight with respect to the total composition, particularly between 7-10%. In a particular embodiment, silk fibroin is in an amount of between 3-20% by weight with respect to the total composition, particularly between 7-10%. In a particular embodiment, sodium hyaluronate is in an amount of between 0.1-10% by weight with respect to the total composition, particularly between 1-4%. In a particular embodiment, chitosan is in an amount of between 1-20% by weight with respect to the total composition, particularly between 10-12%. In a particular embodiment, sodium hydroxide is in an amount of between 1-30% by weight with respect to the total composition, particularly between 12-25% or 15-22%.

In an embodiment, the freeze-dried solid composition further comprises at least one active ingredient as defined above. In some embodiments, the active ingredient is selected from the group consisting of aloe vera, menthol, geranium, lavender, urea, hyaluronic acid, silicon, organic silicon, silk, silica, vitamin A, vitamin B3, vitamin C, vitamin E, *Tephrosia purpurea, Mimosa tenuiflora, Capsicum, Calendula,* micro-algae extract, red algae extract (*Chondrus crispus*), sea fern algae extract, rosehip oil, coconut oil, olive oil, grape oil, cetearyl olivate, sorbitan olivate, *Persea Gratissima* (avocado) oil, phytosterols, *Olea Europaea* (olive) fruit oil, collagen, royal jelly, carnauba wax, active carbon, chamomile, shea butter, white claim (kaolin) and combinations thereof. In a particular embodiment, the active ingredient is selected from the group consisting of aloe vera, menthol, geranium, lavender, urea, hyaluronic acid, silicon, silk, silica, vitamin A, vitamin B3, vitamin C, vitamin E and combinations thereof.

In some embodiments, the composition is for use as a moisturizer and comprises e.g., aloe vera and hyaluronic acid as active ingredients; or the composition is for use as a refresher and comprises e.g., menthol and urea as active ingredients; or the composition is for aromatic use and comprises an aroma e.g., geranium and/or lavender as active ingredients; or the composition is for use as a cleanser and moisturizer and comprises silicon microparticles (cleanser), aloe vera and hyaluronic acid (moisturizer) as active ingredients; or the composition is for use as a moisturizer for very dry skins and comprises avocado oil, aloe vera, hyaluronic acid and calendula oil as active ingredients; or the composition is for use as a brightener and comprises silicon as active ingredient; or the composition is for firmness use and comprises silicon and silk as active ingredients; or the composition is for anti-acne use and comprises vitamin B3 as active ingredient; or the composition is for anti-aging use and comprises vitamin A, vitamin C and vitamin E as active ingredients.

In a particular embodiment, the active ingredient is in an amount of between 0.1-25% by weight with respect to the total composition. Particularly, the active ingredient is a combination of 0.1-5% aloe vera and 0.1-5% hyaluronic acid by weight with respect to the total composition; or a combination of 1-7% menthol and 3-7% urea by weight with respect to the total composition; or is 1-5% aroma (e.g., geranium or lavender) by weight with respect to the total composition; or a combination of 3-8% silicon microparticles, 0.1-5% aloe vera and 0.1-5% hyaluronic acid by weight with respect to the total composition; or a combination of 0.1-5% avocado oil, 0.1-5% aloe vera, 0.1-5% hyaluronic acid and 0.1-5% calendula oil by weight with respect to the total composition; or is 3-8% silicon by weight with respect to the total composition; or a combination of 3-8% silicon and 1-5% silk by weight with respect to the total composition; or is 1-7% vitamin B3 by weight with respect to the total composition; or a combination of 15-25% vitamin C and 1-15% vitamin A, C, E.

In an embodiment, the solid composition has different layers, e.g., a layer to open skin pores, a layer comprising the active ingredient, and a layer to tighten skin pores. For instance, the composition is for use as a cleanser and moisturizer, or as a moisturizer for very dry skins. In a particular embodiment, the active ingredient is a combination of 2-5% rosehip oil, 0.1-5% aloe vera and 1-5% avocado oil by weight with respect to the total composition; or a combination of 0.1-5% avocado oil, 0.1-5% phytosterols and 0.1-5% *Olea Europaea* (olive) fruit oil; or a combination of 3-8% silicon and 0.1-2% vitamin K.

### Dried solid film composition

The dried solid film composition comprises at least one thickening agent as defined herein. In a particular embodiment, the thickening agent is selected from the group consisting of natural polysaccharides, polymers and combinations thereof.

Particularly, the thickening agent is a natural polysaccharide. In a particular embodiment, the natural polysaccharide is selected from the group consisting of alginate, psyllium, cellulose, a gum, hyaluronic salts, and chitosan. Particularly, the gum is gellant gum or xanthan gum.

In an embodiment, the thickening agent is a polymer. In a particular embodiment, the polymer is polyvinyl alcohol (PVA).

In a particular embodiment, the thickening agent is selected from the group consisting of alginate, cellulose, gellant gum, xanthan gum, rhamnose, silk fibroin, sericin, chitosan, carrageenan, dextrin, hyaluronic acid, sodium hyaluronate, hydroxypropyl chitosan, sodium alginate, potassium alginate, propylene glycol alginate, starch, pectin, maltodextrin, methylcellulose, hydroxypropylmethylcellulose, sodium carboxymethylcellulose, polyvinyl alcohol, psyllium and combinations thereof. In a particular embodiment, the thickening agent is alginate, psyllium and/or PVA.

In an embodiment, the dried solid film comprising at least one thickening agent further comprises at least one humectant, pH adjuster and/or inorganic salt as described herein. In an embodiment, the dried solid film comprising at least one thickening agent further comprises at least one humectant and at least one inorganic salt. Particularly, the humectant is glycerol/glycerin and the inorganic salt is sodium chloride.

Accordingly, the composition of the dried solid film can comprise alginate, glycerol/glycerin, psyllium, PVA and/or sodium chloride.

Particularly, the dried solid film composition comprises alginate; or a combination of alginate and sodium chloride; or a combination of alginate and glycerol/glycerin; or a combination of alginate and psyllium; or a combination of alginate and PVA; or a combination of alginate, glycerol/glycerin and sodium chloride; or a combination of alginate, psyllium and sodium chloride; and a combination of alginate, PVA and sodium chloride; or a combination of alginate, glycerol/glycerin and psyllium; or a combination of alginate, glycerol/glycerin, psyllium and sodium chloride; or a combination of alginate, glycerol/glycerin, psyllium and PVA; or a combination of alginate, glycerol/glycerin, psyllium, PVA and sodium chloride.

The present invention also provides a dried solid film composition comprising 100% alginate by weight with respect to the total composition; or a combination of 90-99% alginate and 1-10% psyllium by weight with respect to the total composition; or a combination of 55-85% alginate and 15-45% PVA by weight with respect to the total composition; or a combination of 70-90% alginate, 15-25% PVA and 1-5% sodium chloride by weight with respect to the total composition; or a combination of 65-99.5% alginate and 0.5-35% glycerol/glycerin by weight with respect to the total composition; or a combination of 65-95% alginate, 5-35% glycerol/glycerin and 1-5% psyllium by weight with respect to the total composition; or a combination of 70-50% alginate, 15-25% glycerol/glycerin, 1-5% psyllium and 1-3% sodium chloride by weight with respect to the total composition; or a combination of 45-70% alginate, 10-25% glycerol/glycerin, 1-3% psyllium and 15-35% PVA by weight with respect to the total composition.

In a particular embodiment, alginate is in an amount of between 45-99.9% by weight with respect to the total composition, particularly between 52-99.50%. In a particular embodiment, glycerol/glycerin is in an amount of between 0.1-40% by weight with respect to the total composition, particularly between 0.5-35%. In a particular embodiment, psyllium is in an amount of between 1-10% by weight with respect to the total composition, particularly between 1.5-5%. In a particular embodiment, PVA is in an amount of between 10-40% by weight with respect to the total composition, particularly between 14-33.5%. In a particular embodiment, sodium chloride is in an amount of between 0.1-5% by weight with respect to the total composition, particularly between 1-1.5%.

In an embodiment, the dried solid film composition further comprises at least one active ingredient as described herein, wherein the active ingredient can be particularly an exfoliant material, a moisturizer or a cleanser. In some embodiments, the active ingredient is e.g., aloe vera, menthol, geranium, lavender, urea, hyaluronic acid, silicon, organic silicon, silk, silica, vitamin A, vitamin B3, vitamin C, vitamin E, *Tephrosia purpurea, Mimosa tenuiflora, Capsicum, Calendula,* micro-algae extract, red algae extract (*Chondrus crispus*), sea fern algae extract, rosehip oil, coconut oil, olive oil, grape oil, cetearyl olivate, sorbitan olivate, *Persea Gratissima* (avocado) oil, phytosterols, *Olea Europaea* (olive) fruit oil, collagen, royal jelly, carnauba wax, active carbon, chamomile, shea butter, white claim (kaolin) and combinations thereof. In a particular embodiment, the active ingredient is selected from the group consisting of hyaluronic acid, aloe vera, olive oil, silica, micro-algae extract, red algae extract (*Chondrus crispus*), sea fern algae extract, and combinations thereof.

### Second enveloping element composition

The second enveloping element composition can comprise at least one thickening agent as defined above on the inner side. In a particular embodiment, the thickening agent is selected from the group consisting of natural polysaccharides or monosaccharides, polypeptides and combinations thereof.

Particularly, the thickening agent is a natural polysaccharide. In a particular embodiment, the natural polysaccharide is selected from the group consisting of alginate, cellulose, a gum, hyaluronic salts, and chitosan. Particularly, the gum is gellant gum or xanthan gum.

In an embodiment, the polypeptide is silk fibroin.

In an embodiment, the thickening agent is selected from the group consisting of alginate, cellulose, gellant gum, xanthan gum, rhamnose, silk fibroin, sericin, chitosan, carrageenan, dextrin, hyaluronic acid, sodium hyaluronate, hydroxypropyl chitosan, sodium alginate, potassium alginate, propylene glycol alginate, starch, pectin, maltodextrin, methylcellulose, hydroxypropylmethylcellulose, sodium carboxymethylcellulose, polyvinyl alcohol, psyllium and combinations thereof. In a particular embodiment, the thickening agent is alginate.

The second enveloping element composition can comprise at least one humectant, pH adjuster and/or inorganic salt as defined above on the inner side. In a particular embodiment, the humectant, pH adjuster and/or inorganic salt are glycerin/glycerol, urea, sodium hydroxide and/or sodium chloride.

The second enveloping element comprises at least one synthetic polymer as defined above. In an embodiment, the synthetic polymer is selected from the group consisting of PGA, PGA-PEG, PASP, PASP-PEG, PLA, PLA-PEG, PLGA, PLGA-PEG, PCL, PHAs, poly p-dioxanonse, poly urethane, nylon, poly phosphor esters, poly glycolic acid and combinations thereof. In a particular embodiment, the synthetic polymer is selected from the group consisting of PLA, PCL and combinations thereof.

Particularly, the second enveloping element comprises alginate and PLA; or alginate and PCL; or alginate, PLA and PCL.

In an embodiment, the second enveloping element composition further comprises at least one active ingredient as described herein, wherein the active ingredient can be particularly e.g., aloe vera, menthol, geranium, lavender, urea, hyaluronic acid, silicon, organic silicon, silk, silica, vitamin A, vitamin B3, vitamin C, vitamin E, *Tephrosia purpurea, Mimosa tenuiflora, Capsicum, Calendula,* micro-algae extract, red algae extract (*Chondrus crispus*), sea fern algae extract, rosehip oil, coconut oil, olive oil, grape oil, cetearyl olivate, sorbitan olivate, *Persea Gratissima* (avocado) oil, phytosterols, *Olea Europaea* (olive) fruit oil, collagen, royal jelly, carnauba wax, active carbon, chamomile, shea butter, white claim (kaolin) and combinations thereof. In a particular embodiment, the active ingredient is selected from the group consisting of silicon, aloe vera, hyaluronic acid, active carbon, kaolin, and combinations thereof. Silicon can exhibit a cleansing effect; aloe vera a softening, moisturizing and refreshing effect; hyaluronic acid a moisturizing and anti-aging effect; and active carbon and kaolin, a cleansing effect.

In an embodiment, the outer side of the second enveloping element acts as external packaging and the inner side acts as a face mask with moisturizing or cleansing effects.

### Personal care, packaging and other uses

The present invention also relates to the use of the solid compositions according to the invention and their combinations thereof as a cosmetic product, an oral care product, an edible/nutraceutical product, a dermatological product or a home care product. Further, the invention relates to the solid compositions according to the invention and their combinations thereof for use as a pharmaceutical product.

The present invention also relates to the use of the solid compositions according to the invention and their combinations thereof as a personal care product, that is, a product suitable for a cosmetic, oral care (or dental), dermatological and pharmaceutical use. Non-limiting examples of personal care products are cleansers, moisturizers, make up removers, exfoliants, face masks, skin lotions, hand lotions, suntan lotions, shampoos, conditioners, hair creams, deodorants, make-up or foundations, baby cleansers, depilatory solid cosmetics, medicaments, toothpastes, or mouthwashes. In some embodiments, the personal care function is a cosmetic function selected from the group consisting of cleansing, moisturizing, make up removing, exfoliating, brightening, firming, refreshing, anti-aging and/or anti-wrinkle, and wherein the personal care function of the freeze-dried solid composition and of the dried solid film are complementary to each other.

The present invention discloses a freeze-dried solid composition (also referred herein to as a base) (e.g., Fig. 1A, Fig. 3), a dried solid film (e.g., Fig. 1B, Fig. 4A-B) and an enveloping element (e.g., Fig. 2A, Fig. 6A-B). The personal care product can comprise at least one of said solid compositions. For instance, the dried solid film can be intended to cover the base, thereby acting as a first enveloping element (e.g., Fig. 1C, Fig. 5). In another embodiment, the enveloping element can package the first enveloping element covering the base, thereby acting as a second enveloping element (e.g., Fig. 2B, Fig. 7). The base, the dried solid film and the enveloping element can be considered personal care products as such and they can be used individually. The two latter are also considered external enveloping elements as such; thus, they can also be used individually. For instance, the dried solid film and the enveloping element can be intended to cover any product or substance other than the freeze-dried solid composition (e.g., a tablet, a capsule, powder). They all can also be considered as personal care products when combined. Thus, the personal care functions may vary according to the compositions and their components such as the active ingredients involved in the personal care product.

In an embodiment, the product is a cleanser. Cleansers can be useful for cleansing the body, especially the skin, removing makeup from the skin such as foundation, blush, mascara, eye shadow. Cleansers are gentle to the skin and provide a refreshing feel during use. Cleansers are also unique in their ability to hydrate the skin as a moisturizer and can also be effective for acne prone skin. Thus, the compositions may be applied to the skin and rinsed from the skin with water.

In an embodiment the product is a moisturizer. Moisturizers can be useful for rehydrating and maintaining hydration in skin and mucous membranes. Moisturizers are able of treating topical skin problems like dry, flaking or cracked skin caused by loss of water, or abrasions and redness caused by exposure and rubbing. They have the capacity to hydrate epithelial cells such as comprise the dermis and mucous membranes. Examples of situations where it may be useful is chapped and broken skin, particularly skin which has been exposed to sun, wind, salt water or cold weather, ocular tissue, the mucous membranes of the nasal passages, oral cavity, the vagina and the colon.

In an embodiment, the product is a make-up remover. Makeup removers are intended to help easily remove makeup that has been applied on the skin. They help to remove the applied color and to make sure it easily wipes off using a tissue or other cloth. The purpose of make-up remover compositions or cleaning compositions is essentially to remove all soiling present on the surface of the skin; such soiling consists essentially of pigmented make-up products particles originating from the surrounding atmosphere, and greases originating either from make-up products or possibly from an excess of sebum.

In an embodiment, the product is an exfoliant. Exfoliants are suitable for regular use by a domestic user in removing dead skin cells from an outermost skin surface such as a skin surface of a face, hands or feet. It can be beneficial for removing dry or dull skin, increasing blood circulation, and brightening and improving skin's appearance. Common exfoliant compositions contain an abrasive component as the exfoliating substance, which are mechanical exfoliants. Exfoliants can also be chemical, or a combination thereof. Besides providing the exfoliating effect, it can also help retaining moisture and avoid damaging the active ingredient.

In an embodiment, the product is an anti-wrinkle or an anti-aging formulation. These products address the signs of aging that are already present, including loss of skin firmness, reduced skin elasticity, increased skin dryness, pigmentation disorders such as age spots and uneven pigmentation, loss of radiance and an increase in dullness, formation of fine lines and wrinkles, increased fragility to trauma (creating bruises and rashes), greater translucency (which shows more veins), or increased redness due to blood vessel dilation.

### Freeze-dried solid composition uses

The personal care product according to the present invention comprises a water-soluble base that provides a care function by being dissolved. Such personal care function is carried out by dissolving the base with an aqueous solvent that is soluble in water. The freeze-dried solid composition not necessarily is completely consumed or melt when providing the care function.

For instance, when the care function is cosmetic, dermatological or pharmaceutical, the base is dissolved when entering into contact with the skin of a subject, the dissolution being caused by the friction of the base with the skin and/or by the presence of moisture in the skin. The moisture in the skin can be "natural", that is, the moisture the skin has *per se*, or it can be caused by previous water application on the skin.

The base comprising a thickening agent can have a personal care function *per se.* For instance, the base can comprise alginate, which can act as a skin moisturizer, cleanser or exfoliant. In addition, the base generally contains few compounds, which have a personal care function as such and make the product with less additives, more natural and sustainable.

The base according to the present invention can be a carrier base that includes an active ingredient and said carrier base is adapted to assist in the application of the active ingredient by being dissolved. Therefore, including an active ingredient in the base according to the invention provides the possibility to have a customizable active ingredient in a base, and said base becoming a vehicle for the administration of said active ingredient.

Accordingly, the base can have more than one function, particularly a double personal care function complimentary to each other. For example, the carrier base can act as a moisturizer while the active ingredients present in the base can act as an exfoliant or cleanser.

An active ingredient according to the present invention can be any kind of active ingredient, depending on the application envisaged. The Examples section below exemplifies several possibilities. An active ingredient can range from a molecule having a pharmaceutical effect to an aroma or a compound having a cosmetic effect.

Furthermore, the base can comprise different layers providing different personal care functions. For instance, the base can have three different layers or more, each of them having a different personal care function. Accordingly, a personal care product can be a stratified sponge with different layers, e.g., an exfoliating layer, an active layer comprising an active compound, and a third layer comprising ingredients to tighten skin pores. In an embodiment, the base has different layers which provide different personal care functions.

### Dried solid film uses

The personal care product according to the present invention can comprise a water-soluble dried solid film that provides a care function by being dissolved. Such personal care function is carried out by dissolving, at least in part, the dried solid film with an aqueous solvent that is soluble in water.

The dried solid film comprising a thickening agent can have a personal care function *per se.* For instance, the dried solid film can comprise alginate, polyvinyl alcohol, glycerin, psyllium or a combination thereof, which can act as a skin moisturizer, cleanser or exfoliant. In this case, due to the composition of the dried solid film, the material is more elastic to be easily manipulated by the user.

The dried solid film according to the present invention can further include an active ingredient, and it is adapted to assist in the application of the active ingredient by being dissolved.

The dried solid film can be sealed on its edges to cover at least a product or a substance (e.g., the freeze-dried solid composition of the invention). Consequently, the dried solid film acts as a first enveloping element. In a particular embodiment, the first enveloping element covering the freeze-dried solid composition is sealed by applying pressure and a salt solution. This embodiment of the present invention is further discussed below.

The dried solid film herein provided can also act as a first enveloping element, by covering at least one solid composition. A non-limiting example of the first enveloping element covering the base (e.g., Fig. 5) is depicted in this section. However, it is to be remarked that the first enveloping element can also cover solid compounds other than the freeze-dried solid composition described herein.

The base as defined herein can be wrapped by a first enveloping element (e.g., Fig. 5). Said first enveloping element, which is the dried solid film, is water-soluble and is also adapted to provide a personal care function by being used up, at least in part. That is, the first enveloping element provides a function that is also a care function, therefore cooperating in the care function with the base. The resulting product (the base and the first enveloping element) can have a synergistic effect. For instance, the first enveloping element can provide a particular personal care effect that is enhanced by the personal care effect of the base (e.g., a cleansing effect followed by an exfoliating effect, wherein the exfoliating effect is enhanced after first applying the cleansing effect). Another synergistic effect would be, e.g., being able to reduce the dose of the second active ingredient, after applying an active ingredient previously. Accordingly, in an embodiment, the first enveloping element has a synergistic effect with the wrapped solid composition (e.g., the base). The first enveloping element not necessarily is completely consumed or melt when providing the care function. Fig. 5 shows a base being completely wrapped by the first enveloping element.

For instance, when the personal care function is cosmetic or dermatological, the first enveloping element can have a function of cleansing, exfoliating, sealing, moisturizing or combinations thereof of the skin to which the base and first enveloping element are applied, thereby providing a personal care function cooperating with the personal care function provided by the base according to the invention. Fig. 4B depicts part of a first enveloping element having silica particles on the surface, thereby providing the first enveloping element with an exfoliating care function.

The first enveloping element can have more than one function, particularly a double personal care function, one of said personal care functions being complimentary to the personal care function of the base.

The first enveloping element therefore contains the base. Both first enveloping element and base will have a personal care function. For instance, if the function is cosmetic or dermatological, the base and the first enveloping element will be applied to the skin in a given order. In the case when the first enveloping element has an exfoliating function, the first enveloping element will be applied first to the skin; then the base, having a brightening function, e.g., will be applied.

Moreover, the first enveloping element can comprise an active ingredient that cooperates with the personal care function of the base. The active ingredient of the first enveloping element can be the same as the one of the base or different.

The base is water and humidity free and is naturally preserved, that is, no preservative agents or cryoprotectants are needed to be included in said composition and the components are stable during storage. Further, the first enveloping element covers the base so that the latter is not exposed to environmental insults, thus substantially improving its preservation. Moreover, when an active ingredient is included in the base, the activity and stability of said ingredient is longer than the active ingredient by itself.

The first enveloping element works as a primary packaging and is particularly in direct contact with the base. It is a material that is suitable for use as packaging as it can be easily manipulated by the user, and this is due to the elasticity of the first enveloping element. In a particular embodiment, the first enveloping elements works as a primary packaging.

The composition of the base and the composition of the first enveloping element are particularly selected from the hereinabove mentioned components that are, not only biodegradable substances, but also water-soluble.

Specifically, the base can also comprise lipophilic substances, such as essential oils from plants. These substances have been found in the present application to increase the stability of the active principles the base may contain. Alternatively, the base can also comprise water-based materials or microsphere-based materials.

Specifically, the first enveloping element can also comprise other components suitable to perform a care function complementary to that of the base. For instance, in a dermatological application, when the first enveloping element provides an exfoliating function, the first enveloping element can comprise silica (silicon dioxide) particles.

The first enveloping element can cover at least one solid composition (e.g., the base). In an embodiment, the first enveloping element covers one solid composition. The base can cover different solid compositions, that can have the same or different personal care functions. For instance, one base could act as a make-up remover, another as an exfoliant and the first enveloping element as a moisturizer. Another example could be a first enveloping element covering two bases with different doses (one dose per base), so that it can be administered separately in time. In an embodiment, the first enveloping element covers more than one solid composition.

### Second enveloping element uses

The personal care product according to the present invention can comprise a second enveloping element comprising a biodegradable and synthetic polymeric film with an inner coating made of a thickening agent. The inner coating provides a care function by being dissolved. Such personal care function is carried out by dissolving, at least in part, the second enveloping element with an aqueous solvent that is soluble in water.

Additionally, said second enveloping element is adapted to provide external packaging. The second enveloping element thus provides a barrier function to a product, such as the herein defined. Since said second enveloping element is adapted to provide external packaging, at least the surface in contact with the external environment has to be resistant and not easily used up or consumed when handled or during transportation. Thus, the second enveloping element is synthetic, i.e., it does not dissolve in contact with an external aqueous solvent.

The second enveloping element can have a personal care function *per se.* For instance, it can comprise e.g., an alginate or cellulose coating to confer roughness to the film, which can act as e.g., an exfoliant. It could further contain an active ingredient on the inner side, e.g., vitamin C. Therefore, the second enveloping element can have different personal care functions.

In addition, the second enveloping element can have an external packaging function *per se.* In an embodiment, the second enveloping element is used as a face mask, wherein the inner part of the polymeric film comprises the active composition, whereas the outer part of the film acts as an external packaging. Thus, the personal care product is at the same time an external packaging element, without the need for additional packaging. This feature makes the external packaging element a sustainable personal care product, because there is no need for an additional packaging (it acts as a packaging by itself) and because all components are biodegradable and non-harmful for the environment.

Furthermore, the second enveloping element can also cover solid compounds/compositions other than the first enveloping element described herein. In an embodiment, the second enveloping element can cover at least one solid composition. The purpose of this second packaging, then, is the storage of a series of solid compositions, as the final package of a cosmetic product, i.e., it would be the outer package that would be visible to the user.

For instance, the second enveloping element can cover at least one first enveloping element, containing the base within (e.g., Fig. 2B, Fig. 7). In an embodiment, the personal care product comprises a second enveloping element comprising a biodegradable and synthetic polymeric film with an inner coating made of a thickening agent, the inner coating being adapted to provide a personal care function by being water-soluble, and wherein the second enveloping element covers the first enveloping element.

In a particular embodiment, the personal care product according to the present invention can further comprise a second enveloping element wrapped around the first enveloping element and being biodegradable. The second enveloping element can be further adapted to also provide a care function and/or assist in the application of the active ingredient or said first enveloping element by being used up, at least in part. The second enveloping element, like the first enveloping element, cannot be completely consumed when providing the care function.

Therefore, the second enveloping element can provide different personal care functions that cooperate in the care functions of the wrapped solid compositions (e.g., the base and/or the first enveloping element). Further, the personal care functions of each composition can have a synergistic effect.

The second enveloping element can be composed of different units, particularly three, giving the second enveloping element an envelope-like appearance, such as the one shown in Fig. 2B, Fig. 6A, Fig. 6B and Fig. 7.

The personal care product according to the present invention is particularly composed by natural products. The composition of the base, the composition of the first enveloping element and the composition of the second enveloping element can comprise natural products, particularly polysaccharides and/or plant extracts.

### Particular embodiments of compositions and their uses

In an embodiment, the personal care product is a cosmetic product. In an embodiment, the base as defined hereinabove includes vitamin C as active ingredient and the first enveloping element is an exfoliant. In an embodiment, the base as defined hereinabove includes an exfoliant product in the base and the first enveloping element is a sealer. Another embodiment comprises the base as defined hereinabove with three different layers, having the first layer an exfoliant activity, the second including vitamin C as active ingredient, the third layer including a moisturizing agent. The first enveloping element is a sealer.

In an embodiment, the personal care product is a hair care or body care product. In an embodiment related to hair care, the base as defined hereinabove includes lauryl glucoside as active ingredient, and the first enveloping element comprises provitamin B5 and vitamin E. In an embodiment related to body care, the base as defined hereinabove includes lauryl glucoside as active ingredient and the first enveloping element is an exfoliant. In an embodiment related to a deodorant, the base as defined hereinabove includes triethyl citrate as active ingredient, and the first enveloping element comprises lavanda. In an embodiment, the base as defined hereinabove includes sodium laureth sulfate as active ingredient and the first enveloping element comprises a moisturizing agent. Another embodiment comprises the base as defined hereinabove including lauryl glucoside as active ingredient and the first enveloping element is a sealer.

In an embodiment, the personal care product is an oral care product. In an embodiment related to a dental mouthwash, the base as defined hereinabove comprises chlorhexidine, cetylpyridinium chloride, hexetidine and triclosan as active ingredients while the first enveloping element comprises menthol, stevia, sucralose and fluorine. In an embodiment related to a toothpaste, the base as defined hereinabove comprises fluorine, calcite and foam.

In an embodiment, the personal care product is a pharmaceutical product. In an embodiment related to a pharmaceutical product, sodium diclofenac is the active ingredient in the carrier base as defined hereinabove, while potassium chloride is comprised in the first enveloping element.

In an embodiment, the solid composition is an edible/nutraceutical product. In an embodiment related to a nutraceutical product, the base as defined hereinabove comprises vitamins as active ingredients and sugar (the base being an edible sweet), while the first enveloping element comprises folic acid and folate derivatives. In an embodiment, the nutraceutical product is a food supplement for pregnant women.

In an embodiment, the solid composition is a home care product. In an embodiment related to a home care product, the base as defined hereinabove comprises a disinfectant (e.g., bleach) or a stain remover (e.g., hydrogen peroxide) as active ingredients, while the first enveloping element comprises a detergent or a floor cleaner such as a surfactant.

### General properties and forms of the solid compositions

The present invention relates to a freeze-dried solid composition and a dried solid film composition comprising at least one thickener. The present invention also relates to a first enveloping element (which is the dried solid film) comprising at least one solid composition (e.g., the freeze-dried solid composition), and to a second enveloping element (external packaging) comprising at least one solid composition (e.g., the first enveloping element).

In an embodiment, the freeze-dried solid composition is in the form of a freeze-dried solid, powder, granules, pellets, solid particles, films, aggregates, pieces or any other solid form. Particularly, the freeze-dried solid composition is in the form of a freeze-dried solid.

In an embodiment, the first and second enveloping elements are in the form of a film or a sheet. The term "film" is not limiting as to its form. According to one embodiment, the film is flat. In a particular embodiment, the film is in the form of a sheet. In a particular embodiment, the film has a three-dimensional structure. In another embodiment, the film is in the form of a portion of a sphere, particularly a hemisphere.

In an embodiment, the first and second enveloping elements comprise at least a solid composition, particularly a solid composition in the form of powder, granule, pellets, solid particles, films, aggregates, pieces or any other solid form.

In an embodiment, the first and second enveloping elements envelop at least one solid composition and prevents microbiological contamination of said solid composition. In another embodiment, the first and second enveloping elements keep the solid compositions for a long period of time, since they conserve the humidity. In an embodiment, the first enveloping element makes it possible to keep the solid compositions (e.g., the freeze-dried solid composition) for at least 1 month, 6 months, 1 year, 2 years, 3 years at room temperature without altering said composition.

In an embodiment, the solid compositions of the present invention are homogeneous. In another embodiment, the solid compositions are heterogeneous, e.g., layered or stratified.

In an embodiment, the solid compositions can be handled without any particular precaution.

The presence of at least one thickener in the solid compositions can modify the viscosity of the liquid composition obtained upon dissolving said compositions in an aqueous solution. In a particular embodiment, the dissolution of all or part of the solid compositions in an aqueous solution increases the viscosity of said aqueous solution. Particularly, the liquid composition obtained after dissolving the solid compositions in water has a viscosity ranging from 350 to 4000 cP at 30 °C. According to one embodiment, the dissolution in water of the solid compositions of the invention does not lead to the formation of a gel.

In an embodiment, the solid compositions of the present invention can have different properties, such as rigidity, flexibility, elasticity, viscoelasticity, plasticity or strength. Particularly, the freeze-dried solid composition is rigid. Particularly, the dried solid film is elastic. Particularly, the dried solid film is plastic. Particularly, the dried solid film is flexible. Particularly, the second enveloping element is elastic, resistant and tenacious. In a particular embodiment, the second enveloping element has good mechanical properties, in particular when it is used as packaging, it has good resistance to the operations of forming, filling, transport and conservation. Particularly, the first and second envelopes can have a planar, flexible or semirigid shape, particularly adapted to the shape of the part of the body to which they are intended to be applied.

The freeze-dried solid composition can be flat or three-dimensional. In a particular embodiment, the freeze-dried solid composition is in the form of a flattened sphere. In an embodiment, the dimensions of the freeze-dried solid composition are such that it has a diameter ranging from 0.1 to 10 cm, particularly ranging from 0.5 to 2 cm. In an embodiment, the freeze-dried solid composition has a thickness of 0.1 mm to 10 cm, particularly between 0.5 to 2 cm. In an embodiment, the freeze-dried solid composition is white. In another embodiment, the freeze-dried solid composition comprises a dye.

In an embodiment, the dried solid film is flat. In another embodiment, the dried solid film is sealable in order to form a bag. The bag can be obtained by sealing two sheets of the dried solid film. The bag can also be obtained by sealing a sheet of the dried solid film folded in half. In an embodiment, the dried solid film is in the form of a hollow hemisphere. In an embodiment, the dimensions of the dried solid film are such that it has a size ranging from 0.1 to 10 cm. In a particular embodiment, the dimensions of the dried solid film depend on the dimensions of the wrapped solid composition (e.g., the freeze-dried solid composition). In a particular embodiment, the dried solid film is in the form of a sheet 100 µm to 1 cm thick. In an embodiment, the dried solid film is transparent. In one embodiment, the dried solid film is translucent. In an embodiment, the dried solid film is colorless. In another embodiment, the dried solid film comprises a dye.

In an embodiment, the second enveloping element film is flat. In another embodiment, the second enveloping element is sealable in order to form a bag. The bag can be obtained by sealing two sheets of the polymeric film. The bag can also be obtained by sealing a sheet of the polymeric film folded in half. In an embodiment, the enveloping element makes it possible to improve the presentation of the products which it contains, in particular by the various and attractive shapes which is possible to give to the enveloping element of the invention. The term enveloping element is not limiting as to its form. In one embodiment, the enveloping element is in the form of a sachet, an envelope, a capsule, a capsule or a shell. In a particular embodiment, the enveloping element is in the form of a sachet. The enveloping element can be in the form of a hollow box, sphere or hemisphere. In an embodiment, the inner part of the enveloping element is scratched to increase the specific surface area and consequently the roughness. The scratches can be by using a razor. The presence of scratches helps the interaction between the synthetic polymer and the thickening agent. In an embodiment, the dimensions of the enveloping element composition are such that it has a length of 20 cm and a thickness of 10 cm. In a particular embodiment, the dimensions of the enveloping element depend on the dimensions of the wrapped solid composition (e.g., the dried solid film). In a particular embodiment, the polymeric film is in the form of a sheet 100 µm to 1 cm thick. In an embodiment, the enveloping element is transparent. In one embodiment, the enveloping element is translucent. In an embodiment, the enveloping element is colorless. In another embodiment, the enveloping element comprises a dye.

In a particular embodiment, the size of the first and second enveloping elements depend on the amount and density of the solid compositions to be wrapped. In one embodiment, the envelopes contain an amount of solid composition ranging from 1 g to 100 g, particularly from 1 g to 10 g.

In an embodiment, the solid compositions of the present invention are injectable compositions, i.e., they can be 3D printed to reproduce a specific shape, e.g., an ergonomic shape adaptable to the shape of the hand (e.g., a conic shape). Particularly, the second enveloping element is 3D printed.

### Methods of preparation

The present invention further refers to methods to obtain the compositions as defined herein. The method of preparation can vary according to the particular solid composition to be obtained.

For the preparation of the base, the method comprises preparing a solution of all components in water (e.g., thickening agents and optionally humectants, inorganic salts, pH adjusters, and/or active ingredients); stirring the solution until its homogenization; freezing the solution (e.g., at -20 °C for 48 h); and freeze-drying the frozen solution. In a particular embodiment, sodium hydroxide is added to the solution in suitable amounts to adjust the pH. In another embodiment, the solution is left overnight at room temperature in a shaker. All components of the solid compositions are soluble in water at low temperatures, therefore allowing a better preservation of the active ingredients present in the solid compositions. In another embodiment, the preparation of the solution is performed at a temperature below 40 °C, particularly at room temperature. In an embodiment, the step of freeze-drying is performed at -50 °C at a pressure of 0.050 mPa for 48-72 hours in order to ensure the complete elimination of water content in the solution. More details of the method of preparation of the base are included in Example 1.

For the preparation of the dried solid film, the method comprises preparing a solution of all components in water (e.g., thickening agents and optionally humectants, pH adjusters, inorganic salts and/or active ingredients); placing the solution on a mold; and drying the solution to form a dried film. Particularly, the solution is dried at a temperature below 40 °C, particularly at room temperature. In an embodiment the drying step is performed in a turbulent flow hood for 48-72 hours. More details of the method of preparation of the base are included in Example 3.

The method of covering the base within the first enveloping element comprises the steps of covering the freeze-dried solid composition with the first enveloping element; and sealing the edges of the first enveloping element by applying temperature, a salt solution and/or pressure. In an embodiment, the step of sealing comprises placing a solution of salt in the borders of the first enveloping element (pressure can be applied), particularly comprising 2% by weight of a monovalent ion salt (e.g., sodium chloride or potassium chloride) with respect to the total volume of solution in water. In this way, the first enveloping element is sealed on its borders in a proper way. Particularly the salt is sodium chloride. In a particular embodiment, a temperature up to 60 °C can be applied for sealing the first enveloping element. Fig. 5 shows the first enveloping element sealed and the base contained therein.

Alternatively, the sealing of the first enveloping element to cover the base can be performed in dry conditions, e.g., by applying pressure but without placing any solution in the borders.

The methods for the preparation of the base and/or the first enveloping element can comprise the selection of at least one of the following components: alginate, cellulose, gellant gum, xanthan gum, silk fibroin, sodium hyaluronate, chitosan, glycerin/glycerol, urea, psyllium, rhamnose, sodium hydroxide and/or combinations thereof.

In some embodiments, the base components in the step of preparation of the solution are in an amount of 0.75-3% alginate, 1.5-5% cellulose, 0.2-1% gellant gum, 0.01-1% xanthan gum, 4-6% glycerin, 0.1-5% urea, 1-4.5% sodium hydroxide, 1-5% silk fibroin, 0.1-2% sodium hyaluronate, 2-4% chitosan and 4-6% rhamnose by weight% with respect to the total volume of water.

In some embodiments, the first enveloping element components in the step of preparation of the solution are a mixture of 1-3% alginate and 0.1-5% *Plantago psyllium* seed extract by weight% with respect to the total volume of water.

For the preparation of the second enveloping element, the method comprises a first step of dissolving a synthetic polymer in an organic solvent (e.g., dichloromethane) or heating it at high temperature. Then, the melt polymer or solution is poured onto a e.g., Teflon mold to allow drying of the film. The thickness of the film is controlled by the concentration of the polymer solution as well as by the size of the mold. The polymer is left until full evaporation or complete drying. Particularly, the polymer is left drying at a temperature below 40 °C and more particularly, at room temperature. Once the polymer is completely dry, the surface of the synthetic polymer is scratched to increase the surface area. The thickening agent is then added in solution form, which is left drying overnight until complete drying. The presence of scratches will help the interaction between the synthetic polymer and the thickening agent.

Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. Furthermore, the present invention covers all possible combinations of particular and preferred embodiments described herein. Examples and drawings are provided herein for illustrative purposes, and without intending to be limiting to the present invention.

### EXAMPLES

The present invention will be described with reference to the following examples, but is not limited thereto. The blending amounts are expressed in "% by mass" with respect to the system in which the component is blended unless otherwise specified.

### Example 1: Preparation of different bases which differ in the scaffold (basic composition)

For the preparation of the bases, the components were diluted in order to form a viscous solution. The combination of different materials as shown in Table 1 was used for the preparation of the bases, which define the scaffold for each base. The different components were weighed and diluted in 100 ml distilled water. Sodium hydroxide was added in each solution in suitable amounts to adjust the pH. The solutions were left overnight at room temperature in a shaker.

Once each viscous solution was obtained, it was frozen at -20 °C for 48 hours. Then, each solution was freeze-dried in order to obtain a water-free sponge. For this purpose, frozen viscous solutions were placed in the freeze dryer, which was at -50 °C to avoid thawing of the solution, and it was maintained at this temperature and at a pressure of 0.050 mPa for 48-72 hours in order to ensure the complete elimination of water content in the solution.

**Table 1. Concentrations of the different materials for the preparation of different bases.**

| **Formulation** | **Test examples of bases (blending amount, %)** | | | | | | |
|---|---|---|---|---|---|---|---|
| | **1** | **2** | **3** | **4** | **5** | **6** | **7** |
| Sodium alginate | 34.00 | 30.00 | 32.00 | 42.00 | 15.00 | 72.50 | 47.50 |
| Cellulose | 20.00 | 15.00 | 16.00 | 16.40 | 25.00 | 22.50 | 47.50 |
| Gellant gum | 2.50 | 0.80 | 1.00 | 0.80 | - | - | - |
| Xanthan gum | 0.20 | 0.80 | 1.00 | 0.80 | 0.40 | - | - |
| Glycerin | 10.30 | 17.00 | 18.00 | 20.00 | 29.80 | 5.00 | 5.00 |
| Urea | 1.00 | 17.00 | 18.00 | 20.00 | 29.80 | - | - |
| Rhamnose | 10.00 | 7.70 | - | - | - | - | - |
| Silk fibroin | 10.00 | 7.70 | 10.00 | - | - | - | - |
| Sodium hyaluronate | 1.00 | 4.00 | 4.00 | - | - | - | - |
| Chitosan | 11.00 | - | - | - | - | - | - |

### Results

The seven different bases can be candidates to be adequate bases for the purposes of the present invention. The different materials created a solid product with a suitable texture and form that can be used as a base for different applications without the need of including preservatives. Further, the solid product can be partially or totally consumed which results in an eco-friendly product.

### Example 2: Preparation and evaluation of different freeze-dried compositions (bases) with a common scaffold and different active ingredients

Steps and conditions referring to the production method of the base were performed as described in Example 1, but in this case using the scaffold as defined in Table 2. Active ingredients were also incorporated, which provided a desired effect, as detailed in Table 3. Therefore, different bases were prepared from the same common scaffold, each one with a specific cosmetic or pharmaceutical effect.

**Table 2. Concentrations of different basic materials for the preparation of the base, which define the scaffold for the base.**

| **Formulation** | **Blending amount (%)** |
|---|---|
| Sodium alginate | 15.00 |
| Cellulose | 25.00 |
| Xanthan gum | 0.40 |
| Glycerin | 29.80 |
| Urea | 29.80 |
| Sodium hydroxide | Suitable amount |

**Table 3. Concentrations of different active ingredients of different bases produced from the same common scaffold, and their corresponding effect.**

| **Test examples** | **Active ingredient(s)** | **Blending amount (%)** | **Personal care use** |
|---|---|---|---|
| 1 | Aloe vera | 0.1 | Moisturizing (option 1) |
| | Hyaluronic acid | 0.5 | |
| 2 | Aloe vera | 0.5 | Moisturizing (option 2) |
| | Hyaluronic acid | 0.1 | |
| 3 | Menthol | 2.0 | Refreshing (option 1) |
| | Urea | 5.0 | |
| 4 | Menthol | 5.0 | Refreshing (option 2) |
| | Urea | 5.0 | |
| 5 | Geranium | 2.5 | Aroma |
| 6 | Lavender | 2.5 | Aroma |
| 7 | Silicon microparticles | 5.0 | Layered (2 layers): cleansing + moisturizing |
| | Aloe vera | 0.1 | |
| | Hyaluronic acid | 0.5 | |
| 8 | Avocado oil | 1.0 | Layered (3 layers): moisturizing for very dry skins |
| | Aloe vera | 0.1 | |
| | Hyaluronic acid | 0.5 | |
| | Calendula oil | 1.0 | |
| 9 | Silicon | 6.0 | Brightness |
| 10 | Silicon | 6.0 | Firmness |
| | Silk | 2.0 | |
| 11 | Vitamin B3 | 4.0 | Anti-acne |
| 12 | Vitamin C | 20.0 | Anti-aging (option 1) |
| | Vitamin A, C, E | 1.0 | |
| 13 | Vitamin C | 20.0 | Anti-aging (option 2) |
| | Vitamin A, C, E | 10.0 | |

### Results

Different evaluation methods and criteria were used to test the bases. After preparing the different bases according to their active ingredients, they were evaluated as such (Table 4), and also after rehydration and application to the skin (Table 5).

The parameters used to evaluate the bases as such were texture (smooth, soft, rigid), odor (yes, no), brightness (yes, no), dissolution (not entirely, medium, rapid, very rapid; and its associated percentage), thickness (low, medium, high), viscosity (no, low, yes) and the homogeneity to determine the spreading capacity once rehydrated (poor, regular, good, very good).

The parameters used to evaluate the effect of the bases on the skin after rehydration with water were absorption (rapid, very rapid), oiliness (no, low), stickiness (no, low, high), freshness (no, low, yes), softness (no, yes), immediate effect on the skin (pleasant, lifting, itching, mask effect, among others), and effect at 12 hours (hydration, good, pleasant, sticky, pealing effect, among others).

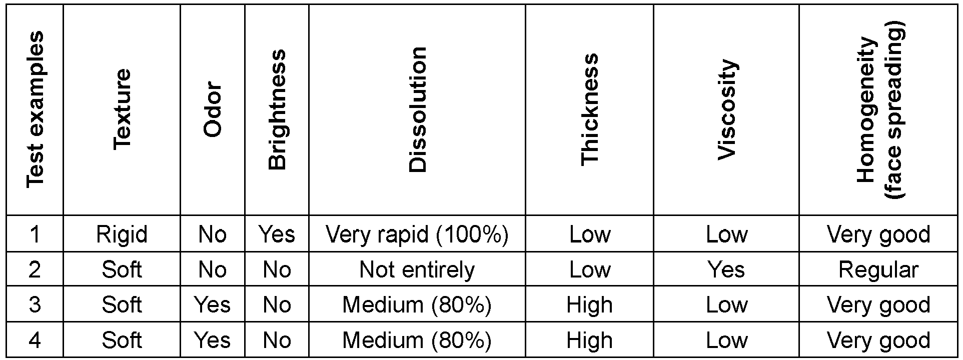

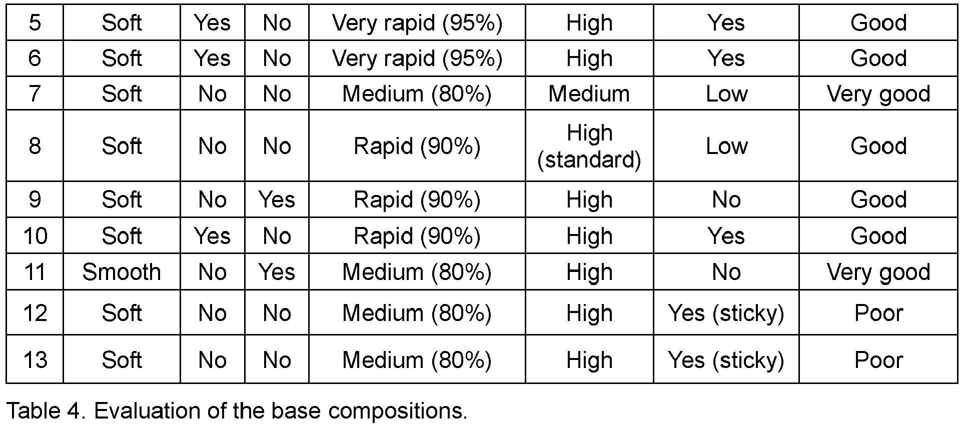

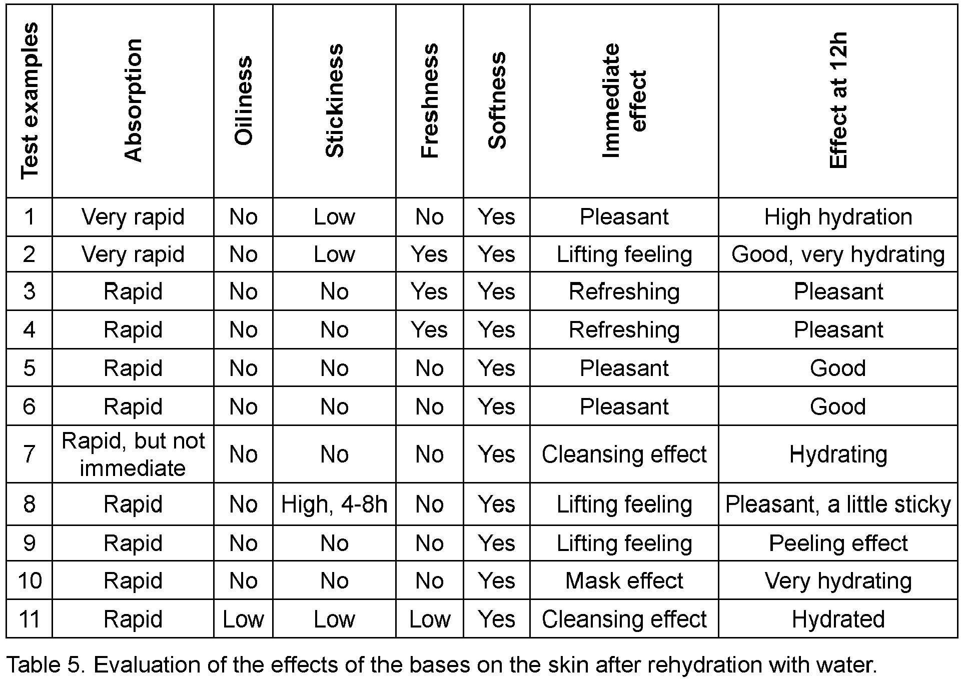

The rehydrated bases provided a wide variety of effects on the skin. For instance, the bases with silicon microparticles provided a cleansing effect while the bases with oils or moisturizing ingredients ended in a very hydrated skin. Therefore, this suggest that a great diversity of active ingredients can be added to the base and provide the desired effect.

### Example 3: Preparation of the dried solid film (first enveloping element)

For the preparation of the first enveloping element, the combination of sodium alginate 1-3% (w/v) and other materials indicated in Table 6 (e.g., *Plantago psyllium* seed extract 0.1-5% (w/v)) were weighed and dissolved in distilled water. A 5% silica was added in each formulation, and all formulations were tested with and without silica. Once a homogeneous viscous solution was obtained, the solution was placed on a mold and left drying at room temperature in a turbulent flow hood for 48-72 hours, until a dried film was obtained.

To complete the sealing of the first enveloping element around the base, a solution of 2% NaCl was placed on the borders of the film and sealing was performed by applying pressure.

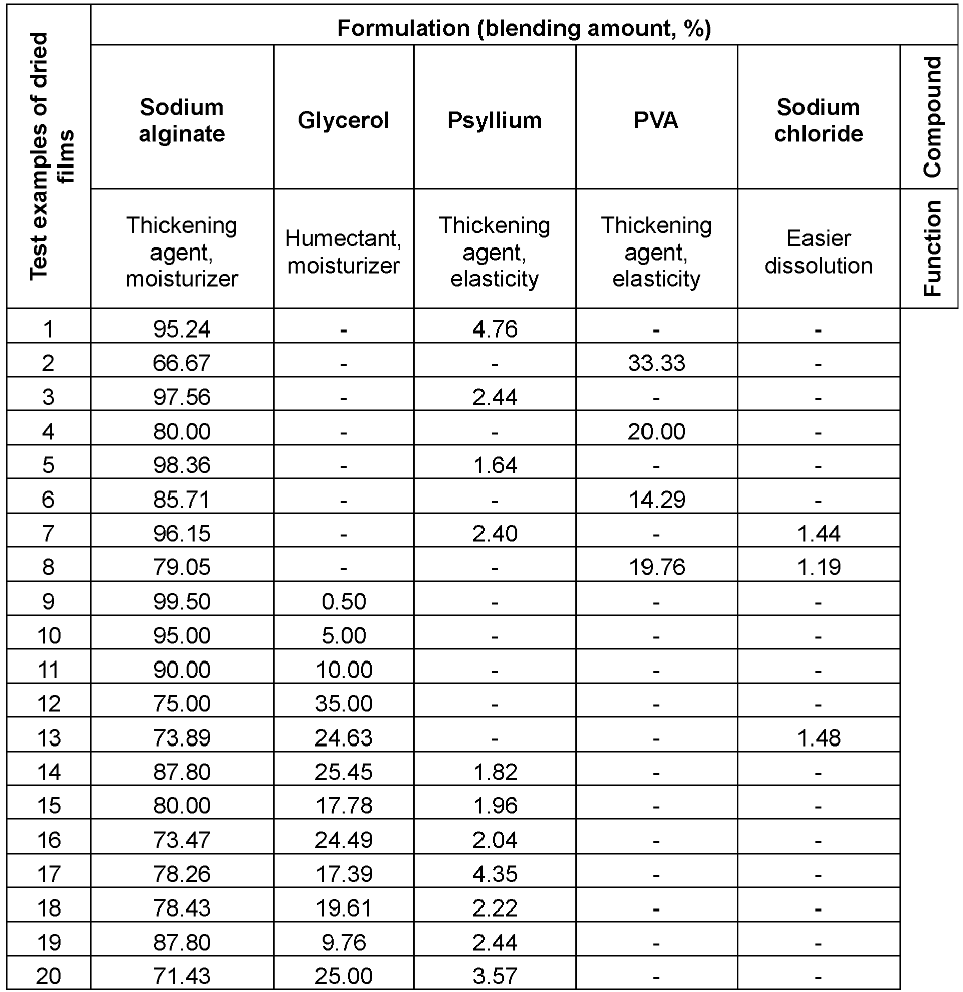

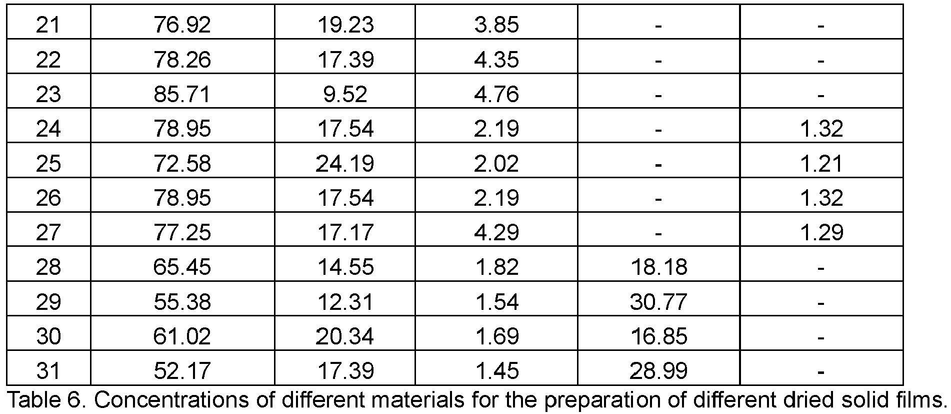

### Results

Dried solid films with different mechanical properties were obtained, being adequate for the purposes of the present invention. Viscosity of the film increased with high concentrations of alginate. In the same lines, elasticity increased with the incorporation of glycerol, PVA or psyllium. Glycerol was added in order to obtain a more flexible and homogenous film. PVA in higher amounts than psyllium provided a fast dissolution and also an increase in the elastic properties of the films. The addition of sodium chloride in low amounts facilitated the dissolution. Therefore, depending on the composition used, different dried solid films can be obtained, providing a wide variety of products for different applications.

### Example 4: Preparation of the enveloping element (external packaging)

### Production method

Two different enveloping elements were prepared, using sodium alginate as thickening agent (inner side), and PCL or PLA as synthetic polymer (Table 7). The synthetic polymer PCL or PLA was dissolved in dichloromethane (organic solvent), poured onto a Teflon mold and left drying at room temperature until full evaporation or complete drying. Then, the inner side of the formed film was scratched to increase the surface area. Finally, sodium alginate was added in a solution form on the scratched inner part of the film and left drying overnight.

**Table 7. Concentrations of different materials for the preparation of the enveloping element.**

| **Formulation** | **Blending amount (%)** | |
|---|---|---|
| Test examples | 1 | 2 |
| **Inner side** | | |
| Sodium alginate | 100 | 100 |
| **Outer side** | | |
| PCL | 100 | - |
| PCA | - | 100 |

### Results

The different materials created a solid product with suitable properties to be used as an external packaging as well as a personal care product, since multiple active ingredients or other components can be added into it to obtain the desired effect.

## Claims

1. A personal care product adapted to provide a care function by being water-soluble, comprising:
- a freeze-dried solid composition comprising at least one thickening agent, the composition adapted to provide a personal care function by being water-soluble; and
- a dried solid film comprising at least one thickening agent, the film adapted to provide a personal care function by being water-soluble,
wherein the dried solid film acts as a first enveloping element covering the freeze-dried solid composition.

2. The product according to claim 1, wherein the personal care function is provided upon contact and/or friction with an aqueous solvent at room temperature.

3. The product according to any of claims 1-2, wherein the personal care function is a cosmetic function selected from the group consisting of cleansing, moisturizing, make up removing, exfoliating, brightening, firming, refreshing, anti-aging and/or anti-wrinkle, and wherein the personal care function of the freeze-dried solid composition and of the dried solid film are complementary to each other.

4. The product according to any of claims 1-3, wherein the thickening agent is a polysaccharide which is selected from the group consisting of a gum, a natural polysaccharide, a cellulose, a cellulose derivative and a starch.

5. The product according to any of claims 1-3, wherein the thickening agent is selected from the group consisting of alginate, cellulose, gellant gum, xanthan gum, rhamnose, silk fibroin, sericin, chitosan, carrageenan, dextrin, hyaluronic acid, sodium hyaluronate, hydroxypropyl chitosan, sodium alginate, potassium alginate, propylene glycol alginate, starch, pectin, maltodextrin, methylcellulose, hydroxypropylmethylcellulose, sodium carboxymethylcellulose, polyvinyl alcohol, and psyllium.

6. The product according to any of claims 1-5, wherein the freeze-dried solid composition and/or the dried solid film further comprise at least one component selected from the group consisting of a humectant, an inorganic salt, and a pH adjuster.

7. The product according to claim 6, wherein the humectant is selected from the group consisting of glycerin/glycerol, urea, propylene glycol, 1,3-propanediol, honey, sorbitol, glucose syrup, polysaccharide syrup, and monosaccharide syrup.

8. The product according to any of claims 6-7, wherein the inorganic salt is selected from the group consisting of sodium chloride, calcium chloride, magnesium chloride, sodium bicarbonate, potassium chloride, sodium sulfate, calcium carbonate, and calcium phosphate.

9. The product according to any of claims 1-8, wherein the freeze-dried solid composition comprises alginate; or a combination of alginate and cellulose; or a combination of alginate, cellulose and glycerin/glycerol; or a combination of alginate, cellulose, glycerin/glycerol and urea; or a combination of alginate, cellulose, glycerin/glycerol, urea and xanthan gum; or a combination of alginate, cellulose, glycerin/glycerol, urea and gellant gum; or a combination of alginate, cellulose, glycerin/glycerol, urea, xanthan gum and gellant gum; or a combination of alginate, cellulose, glycerin/glycerol, urea, xanthan gum, gellant gum, and silk fibroin; or a combination of alginate, cellulose, glycerin/glycerol, urea, xanthan gum, gellant gum, silk fibroin, and sodium hyaluronate; or a combination of alginate, cellulose, glycerin/glycerol, urea, xanthan gum, gellant gum, silk fibroin, sodium hyaluronate, and rhamnose; or a combination of alginate, cellulose, glycerin/glycerol, urea, xanthan gum, gellant gum, silk fibroin, sodium hyaluronate, rhamnose, and chitosan.

10. The product according to any of claims 1-9, wherein the dried solid film comprises alginate; or a combination of alginate and sodium chloride; or a combination of alginate and glycerol/glycerin; or a combination of alginate and psyllium; or a combination of alginate and polyvinyl alcohol; or a combination of alginate, glycerol/glycerin and sodium chloride; or a combination of alginate, psyllium and sodium chloride; and a combination of alginate, polyvinyl alcohol and sodium chloride; or a combination of alginate, glycerol/glycerin and psyllium; or a combination of alginate, glycerol/glycerin, psyllium and sodium chloride; or a combination of alginate, glycerol/glycerin, psyllium and polyvinyl alcohol; or a combination of alginate, glycerol/glycerin, psyllium, polyvinyl alcohol and sodium chloride.

11. The product according to any of claims 1-10, wherein the freeze-dried solid composition and/or the dried solid film further comprises at least one active ingredient selected from the group consisting of aloe vera, menthol, geranium, lavender, urea, hyaluronic acid, silicon, organic silicon, silk, silica, vitamin A, vitamin B3, vitamin C, vitamin E, *Tephrosia purpurea, Mimosa tenuiflora, Capsicum, Calendula,* micro-algae extract, red algae extract, sea fern algae extract, rosehip oil, coconut oil, olive oil, grape oil, cetearyl olivate, sorbitan olivate, *Persea Gratissima* oil, phytosterols, *Olea Europaea* fruit oil, collagen, royal jelly, carnauba wax, active carbon, chamomile, shea butter, and white claim.

12. The product according to any of claims 1-11, further comprising a second enveloping element comprising a biodegradable and synthetic polymeric film with an inner coating made of a thickening agent, the inner coating being adapted to provide a personal care function by being water-soluble, and wherein the second enveloping element covers the first enveloping element.

13. The product according to claim 12, wherein the synthetic polymeric film comprises a polymer selected from the group consisting of polylactic acid, polyglutamic acid, poly caprolactone, poly(aspartic acid), poly(lactic-co-glycolic acid), polyhydroxyalkanoate, poly(p-dioxanonse), polyurethane, nylon, polyphosphor ester, and polyglycolic acid.

14. The product according to any of claims 12-13, wherein the second enveloping element is used as a face mask.

15. A method to prepare a personal care product comprising the steps of:
a) preparing a freeze-dried solid composition comprising the steps of:
i) preparing a solution of at least one thickening agent and water;
ii) stirring the solution until its homogenization;
iii) freezing the solution; and
iv) freeze-drying the frozen solution;
b) preparing a dried solid film comprising the steps of:
i) preparing a solution of at least one thickening agent and water;
ii) placing the solution on a mold; and
iii) drying the solution to form a dried film;
c) covering the freeze-dried solid solution with the dried solid film; and
d) sealing the edges of the dried solid film by applying temperature, pressure and/or a salt solution.
